# EUROPEAN PATENT APPLICATION

(11) **EP 4 792 738 A1**
(43) Date of publication of application: **19.08.2026**
(21) Application number: 24879683.1
(22) Date of filing: 11.10.2024
(51) Int. Cl.: A61B 5/021, A61B 5/026

(54) **CIRCULATORY SYSTEM PHYSIOLOGICAL INDEX CALCULATION DEVICE, COMPUTER PROGRAM, AND RECORDING MEDIUM**

(30) Priority: 15.10.2023 JP 2023177941
(71) Applicant: Delta Tooling Co., Ltd., Hiroshima-shi, Hiroshima 736-0084 (JP)
(72) Inventor: FUJITA, Etsunori, Hiroshima-shi, Hiroshima 736-0084 (JP)
(74) Representative: Hoffmann Eitle
(86) International application number: PCT/JP2024/036562
(87) International publication number: WO 2025/084258

(57) **Abstract**

There is provided an art for finding a circulatory system physiological index serving as an index for more accurate estimation of blood pressure. A power spectrum found through a frequency analysis of time-series data of instantaneous heart rate variability is plotted and displayed on a log-log graph. On the plot display, a plurality of types of analysis-target frequency bands are set, and two types of fitted curves of different degrees are fit. As the fitted curves, a plurality of curves each represented by a quartic polynomial or a cubic polynomial having the smallest standard deviation of residuals with respect to the plot display of the power spectrum are found. A fitted curve with a smaller standard deviation of residuals has a higher correlation between the frequency of its inflection point and blood pressure. Therefore, based on this, a fitted curve having a higher correlation with blood pressure is selected. The standard deviation of residuals is also calculated for tangents at inflection points and a straight line between inflection points of the plurality of fitted curves, and one with the smallest standard deviation of residuals is adopted.

## Description

### Technical Field

The present invention relates to an art for finding a circulatory system physiological index correlating with blood pressure and blood flow volume.

### Background Art

In Patent Document 1, the present applicant has disclosed a blood pressure estimation device that analyzes biological signal data obtained from a biological signal detection sensor including a three-dimensional knitted fabric, captures information on vibration generated in a living body by blood flow variability in a ventricular filling phase to an isovolumetric contraction phase, finds an index indicating its fluctuation using the Lorenz plot method, and estimates a blood pressure value from the index (Patent Document 1).

Specifically, this is an art that obtains a biological signal (Acoustic Pulse Wave: APW) that the biological signal detection sensor captures from the dorsal surface, filters its time-series waveform at a predetermined frequency band, obtains a filtered waveform in which the cardiac cycle is manifested, collates this filtered waveform with simultaneously measured electrocardiogram waveform data obtained from an electrocardiograph, identifies waveform components in the ventricular filling phase to the isovolumetric contraction phase, thereafter, sets, as a reference, a gradient of a group of many points that are plotted in a predetermined measurement time using the Lorenz plot method, newly constructs a time-series waveform of an angle difference between the reference gradient and a gradient of a point group in a shorter measurement time, frequency-analyzes the time-series waveform, displays the frequency analysis result on a log-log scale, finds a regression line in a VLF to LF range on the analyzed waveform, defines a gradient of the regression line as a fractal slope, and estimates blood pressure from the fractal slope.

With the focus on the fact that pulse wave velocity (PWV) depends on blood pressure and arterial elasticity, Patent Document 2 discloses an art that measures blood pressure on the premise that blood pressure is proportional to PWV. It further describes that PWV is found from PAT (Pulse arrival time). Since PWV is in inverse proportion to the derivative of PAT, this art estimates blood pressure by finding the derivative of PAT. It further describes that heart rate and oxygen saturation are measured with an optical sensor used in mobile computers such as mobile phones.

Patent Documents 1 and 2 are common in that they are both arts capable of estimating blood pressure without using a blood pressure monitor that uses a cuff, that is, a brachial blood pressure monitor.

### Prior Art Document

### Patent Document

Patent Document 1: Japanese Laid-open Patent Publication No. 2019-122502
Patent Document 2: US Patent Publication No. 2015/0320328A1
Patent Document 3: Japanese Laid-open Patent Publication No. 2022-71786
Patent Document 4: Japanese Laid-open Patent Publication No. 2022-71812

### Non-Patent Document

Non-Patent Document 1: Guyton and Hall Textbook of Medical Physiology, Original Article, 13th Edition, published March 20, 2018, Author: John E. Hall, General Translation Supervisor: Yoshihiro Ishikawa et al., Publisher: Elsevier Japan KK

### Summary of the Invention

### Problems to Be Solved by the Invention

The complex control for maintaining blood pressure homeostasis has fluctuation due to energy variation. Heart rate (HR) is derived from RRI on an electrocardiogram (ECG), heart rate variability (HRV) has fluctuation, and a fluctuation index is calculated from the result of a frequency analysis of heart rate variability. Heart rate variability is reflected in afterload and baroreceptor reflex, and arterial pressure varies due to afterload variation and baroreceptor reflex.

In Patent Document 1, the abovementioned fractal slope is used as the index indicating this fluctuation, but the fractal slope is an index indicating the characteristics of the analyzed waveform only by using the regression line. Therefore, it is difficult to say that this fully reflects the characteristics of blood pressure whose control mechanism is a complex control structure with complexity as described above.

Further, as in Patent Document 2, it is also practiced to detect the pulse wave velocity to find blood pressure. Though it is said that the pulse wave velocity correlates with blood pressure, it cannot be said that their correlation level is very high. Therefore, even if blood pressure is estimated simply based on the pulse wave velocity or its substitute, i.e., pulse wave propagation time, there is a concern about accuracy.

Note that Patent Documents 3 and 4 give a description regarding finding an apex beat wave (CAB) from APW measured from the cardiac apex. Further, Non-Patent Document 1 gives a description regarding autoregulation.

The present invention has been made in consideration of the above, and has an object to provide an art that finds a circulatory system physiological index serving as an index for more accurate estimation of blood pressure without using a cuff. Means for Solving the Problems

To solve the above problem, a circulatory system biological index calculation device of the present invention is a circulatory system physiological index calculation device that finds a circulatory system physiological index correlating with blood pressure and blood flow volume, by using at least one piece of biological signal data out of heart rate and an acoustic pulse wave that is caused by left ventricular pressure variation and obtained from a body surface, the circulatory system physiological index calculation device including:
a biological signal receiving unit that receives the biological signal data;
a time-series variation data calculation unit that finds time-series variation data in which information on time-series variation in the biological signal data is reflected;
a frequency analysis unit that plots and displays a power spectrum found through a frequency analysis of the time-series variation data, on a log-log graph;
a fitted curve computation unit that fits n-th degree polynomials or (n-1)-th degree polynomials to a plurality of types of analysis-target frequency bands set in a range from VLF to LF on the plot display of the power spectrum, and in the analysis-target frequency bands, finds a plurality of fitted curves represented by the n-th or (n-1)-th degree polynomials with the smallest standard deviations of residuals with respect to the plot display of the power spectrum;
a frequency gradient calculation unit that calculates a frequency gradient of a tangent at an inflection point of each of the fitted curves, and when the fitted curve has two inflection points, calculates a frequency gradient of a straight line between the two inflection points; and
an index decision unit that, regarding the tangents at the inflection points or the straight line between the inflection points, calculates standard deviations of residuals with respect to the plot display of the power spectrum, compares the standard deviations of the residuals to select the tangent or the straight line with the smallest standard deviation of the residuals, and decides the frequency gradient of the selected tangent or straight line as the circulatory system physiological index.

Preferably, the analysis-target frequency bands set by the fitted curve computation unit are within a range up to 0.08 Hz from a frequency of any of power spectrum calculation points that are in a 0.006 to 0.034 Hz range including 0.017 Hz, which is a fundamental frequency determined based on a basic period, the basic period being time required for acute autoregulation of blood flow to return blood flow volume to a normal level when blood pressure rises.

Preferably, the fitted curve computation unit determines whether a histogram of the residuals between the fitted curve drawn in each of the analysis-target frequency bands and the plot display of the power spectrum follows a normal distribution, and if the histogram does not follow the normal distribution, adjusts the analysis-target frequency band and re-finds the fitted curve.

Preferably, the n-th degree polynomial is a quartic polynomial, and the (n-1)-th degree polynomial is a cubic polynomial.

Preferably, the fitted curve computation unit:
sets the analysis-target frequency bands within a range up to 0.08 Hz from a frequency of any of power spectrum calculation points that are in a range of 0.006 to 0.034 Hz, and finds a first fitted curve represented by the cubic polynomial and a second fitted curve represented by the quartic polynomial, and
finds a third fitted curve represented by the cubic polynomial in a range up to 0.08 Hz from a frequency of a lower frequency-side inflection point out of two inflection points that are found from the second fitted curve of the quartic polynomial.

Preferably, the index decision unit selects the straight line with the smallest standard deviation of the residuals with respect to the plot display of the power spectrum, out of a total of five straight lines consisting of:
a tangent at an inflection point of the first fitted curve;
tangents at the two inflection points of the second fitted curve;
a straight line between the two inflection points of the second fitted curve; and
a tangent at an inflection point of the third fitted curve.

Preferably, the index decision unit:
finds the standard deviation of the residuals regarding the tangent at the inflection point of each of the first fitted curve, the second fitted curve, and the third fitted curve in a predetermined range, of the plot display of the power spectrum, centered at the inflection point; and
regarding the second fitted curve, further finds the standard deviation of the residuals in a predetermined range, of the plot display of the power spectrum, centered at a midpoint of the straight line between the two inflection points.

A computer program of the present invention is a computer program causing a computer to function as a circulatory system physiological index calculation device that finds a circulatory system physiological index correlating with blood pressure and blood flow volume, by using at least one piece of biological signal data out of heart rate and an acoustic pulse wave that is caused by left ventricular pressure variation and obtained from a body surface, the program including:
a procedure for receiving the biological signal data;
a procedure for finding time-series variation data in which information on time-series variation in the biological signal data is reflected;
a procedure for plotting and displaying a power spectrum found through a frequency analysis of the time-series variation data, on a log-log graph;
a procedure for fitting n-th degree polynomials or (n-1)-th degree polynomials to a plurality of types of analysis-target frequency bands set in a range from VLF to LF on the plot display of the power spectrum, and in the analysis-target frequency bands, finding a plurality of fitted curves represented by the n-th or (n-1)-th degree polynomials with the smallest standard deviations of residuals with respect to the plot display of the power spectrum;
a procedure for calculating a frequency gradient of a tangent at an inflection point of each of the fitted curves, and when the fitted curve has two inflection points, calculating a frequency gradient of a straight line between the two inflection points; and
a procedure for, regarding the tangents at the inflection points or the straight line between the inflection points, calculating standard deviations of residuals with respect to the plot display of the power spectrum, comparing the standard deviations of the residuals to select the tangent or the straight line with the smallest standard deviation of the residuals, and deciding the frequency gradient of the selected tangent or straight line as the circulatory system physiological index.

Preferably, the analysis-target frequency bands are set within a range up to 0.08 Hz from a frequency of any of power spectrum calculation points that are in a 0.006 to 0.034 Hz range including 0.017 Hz, which is a fundamental frequency determined based on a basic period, the basic period being time required for acute autoregulation of blood flow to return blood flow volume to a normal level when blood pressure rises.

Preferably, it is determined whether a histogram of the residuals between the fitted curve drawn in each of the analysis-target frequency bands and the plot display of the power spectrum follows a normal distribution, and if the histogram does not follow the normal distribution, the analysis-target frequency band is adjusted and the fitted curve is re-found.

Preferably, the n-th degree polynomial is a quartic polynomial, and the (n-1)-th degree polynomial is a cubic polynomial.

Preferably, the analysis-target frequency bands are set within a range up to 0.08 Hz from a frequency of any of power spectrum calculation points that are in a range of 0.006 to 0.034 Hz, and a first fitted curve represented by the cubic polynomial and a second fitted curve represented by the quartic polynomial are found, and
a third fitted curve represented by the cubic polynomial is found in a range up to 0.08 Hz from a frequency of a lower frequency-side inflection point out of two inflection points that are found from the second fitted curve represented by the quartic polynomial.

Preferably, the straight line with the smallest standard deviation of the residuals with respect to the plot display of the power spectrum is selected, out of a total of five straight lines consisting of:
a tangent at an inflection point of the first fitted curve;
tangents at the two inflection points of the second fitted curve;
a straight line between the two inflection points of the second fitted curve; and
a tangent at an inflection point of the third fitted curve, and
a frequency gradient of the selected tangent or straight line is decided as the circulatory system physiological index.

Preferably, the standard deviation of the residuals regarding the tangent at the inflection point of each of the first fitted curve, the second fitted curve, and the third fitted curve is found in a predetermined range, of the plot display of the power spectrum, centered at the inflection point; and
regarding the second fitted curve, the standard deviation of the residuals is found within a predetermined range, of the plot display of the power spectrum, centered at a midpoint of the straight line between the two inflection points.

Further, the present invention provides a computer-readable recording medium in which the above computer program is recorded.

### Effect of the Invention

According to the present invention, time-series data of variation in at least one piece of biological signal data out of heart rate and an acoustic pulse wave that is caused by left ventricular pressure variation and obtained from a body surface is frequency-analyzed, the resultant power spectrum is plotted and displayed on a log-log graph, a plurality of types of analysis-target frequency bands are set on the plot display, and two types of fitted curves of different degrees are fit. As the fitted curves, a plurality of curves represented by n-th degree polynomials or (n-1)-th degree polynomials with the smallest standard deviations of residuals with respect to the plot display of the power spectrum are found. A fitted curve having a smaller standard deviation of residuals has a higher correlation between the frequency of its inflection point and blood pressure. Therefore, based on this, a fitted curve having a higher correlation with blood pressure is selected. The standard deviations of residuals are also calculated for tangents at inflection points and a straight line between inflection points of a plurality of fitted curves, and one with the smallest standard deviation of residuals is adopted. As a result, the finally selected frequency gradient has a higher correlation with blood pressure, and in the present invention, this is used as a circulatory system physiological index. According to the present invention, it is possible to obtain a circulatory system physiological index highly correlating with blood pressure to improve the accuracy of blood pressure estimation.

### Brief Description of Drawings

[FIG. 1] FIG. 1 is a block diagram illustrating the configuration of a circulatory system physiological index calculation device according to one embodiment of the present invention.
[FIG. 2] FIG. 2 is a flowchart illustrating a process up to the creation of a power spectrum (spectrum plot) as a frequency analysis result after time-series variation data (Δθi function) indicating heart rate (instantaneous heart rate) variability is frequency-analyzed.
[FIG. 3] FIG. 3 is a chart in which data output in the steps in FIG. 2 are illustrated along the flowchart in FIG. 2.
[FIG. 4] FIG. 4 is a flowchart illustrating a process of obtaining a Δθi function using APW.
[FIG. 5] FIG. 5 is a chart in which data output in the steps in FIG. 4 are illustrated along the flowchart in FIG. 4.
[FIGs. 6] FIG. 6(a) is a scatter plot in which frequencies of inflection points of a fitted curve, which is represented by a quartic polynomial, created using data from a total of 171 subjects (260 cases) consisting of a healthy subject group (95 individuals) and an outpatient group (76 individuals) are plotted in relation to systolic blood pressure in experimental examples, and FIG. 6(b) is a similar scatter plot when an analysis-target frequency band is set to 0.006 to 0.08 Hz.
[FIG. 7] FIG. 7 is an explanatory flowchart of an arithmetic process in a fitted curve computation unit, a frequency gradient calculation unit, and an index decision unit.
[FIGs. 8] FIGs. 8(a) to (c) are charts illustrating examples of data that are obtained when fitted curves are found and circulatory system physiological indexes (GT (GI)) are found in the experimental examples.
[FIGs. 9] FIGs. 9(a) to (c) are charts illustrating examples of data that are obtained when fitted curves are found and circulatory system physiological indexes (GT/GI) are found in the experimental examples.
[FIGs. 10] FIGs. 10(a) to (c) are charts illustrating examples of data that are obtained when fitted curves are found and circulatory system physiological indexes (GT (GI)) are found in the experimental examples.
[FIGs. 11] FIGs. 11(a) to (c) are charts illustrating examples of data that are obtained when fitted curves are found and circulatory system physiological indexes (GT (GI)) are found in the experimental examples.
[FIGs. 12] FIGs. 12(a) to (c) are charts illustrating examples of data that are obtained when fitted curves are found and circulatory system physiological indexes (GT (GI)) are found in the experimental examples.
[FIGs. 13] FIGs. 13(a) to (c) are charts illustrating examples of data that are obtained when fitted curves are found and circulatory system physiological indexes (GT (GI)) are found in the experimental examples.
[FIGs. 14] FIG. 14(a) is a chart illustrating a correlation between values of frequency gradients (GT (GI)) found for all 171 subjects (260 cases) and their systolic blood pressure values (SBP) measured with a brachial blood pressure monitor, and FIG. 14(b) is a cross-tabulation table showing a relationship between the systolic blood pressures and GT (GI).
[FIGs. 15] FIG. 15(a) is a flowchart illustrating a process of calculating an estimated blood pressure using a frequency gradient (GT or GI), which is a circulatory system physiological index, FIG. 15(b) is a chart illustrating a correlation between estimated blood pressure values and measured blood pressure values, and FIG. 15(c) is a cross-tabulation table thereof.
[FIGs. 16] FIG. 16(a) is a three-dimensional response surface created using data of the healthy subject group (95 individuals), and FIG. 16(b) is a three-dimensional response surface created using data of an outpatient group (76 individuals) having heart disease and risk factors.
[FIGs. 17] FIGs. 17(a) to (c) are charts illustrating cross-sections of a three-dimensional response surface where the data of the healthy subject group (95 individuals) and the data of the outpatient group (76 individuals) are combined.
[FIGs. 18] FIG. 18(a) is a chart illustrating a correlation between estimated blood pressure values (eSBP), which are derived using past one-year data of instantaneous heart rates of a 34-year-old (healthy) subject, and his brachial systolic blood pressure values measured when the instantaneous heart rates are measured, and FIG. 18(b) is a cross-tabulation table showing a relationship between the estimated blood pressure values (eSBP) and the measured brachial systolic blood pressure values (SBP).
[FIGs. 19] FIG. 19(a) is a chart illustrating a correlation between estimated blood pressure values (eSBP) derived using past one-year data of instantaneous heart rates of a 55-year-old subject (outpatient: under medication for hypertension), and his brachial systolic blood pressure values measured when the instantaneous heart rates are measured, and FIG. 19(b) is a cross-tabulation table illustrating a relationship between the estimated blood pressure values (eSBP) and the measured brachial systolic blood pressure values (SBP).
[FIGs. 20] FIG. 20(a) is a chart illustrating a correlation between estimated blood pressure values (eSBP), which are derived using past one-year data of instantaneous heart rates of a 67-year-old subject (outpatient: under medication for hypertension), and his brachial systolic blood pressure values measured when the instantaneous heart rates are measured, and FIG. 20(b) is a cross-tabulation table showing a relationship between the estimated blood pressure values (eSBP) and the measured brachial systolic blood pressure values (SBP).
[FIGs. 21] FIGs. 21(a) to (b) are charts for explaining application to a left ventricular pressure waveform.
[FIG. 22] FIG. 22 is a chart for explaining the usefulness of an approximation expression of the left ventricular pressure waveform.
[FIG. 23] FIG. 23 is a chart for explaining reasons why GI often results in a case of normotension and GT often results in cases of hypertension and hypotension.
[FIG. 24] FIG. 24, similarly to FIG. 23, is a chart for explaining reasons why GI often results in a case of normotension and GT often results in cases of hypertension and hypotension.
[FIGs. 25] FIG. 25(a) is an image chart illustrating heart rate variability, blood pressure variability, and fluctuation variability, and FIG. 25(b) illustrates Lorenz plots created based on FIG. 25(a) regarding blood pressure variability and heart rate variability.

### Modes for Carrying out the Invention

### (Circulatory System Physiological Index Calculation Device)

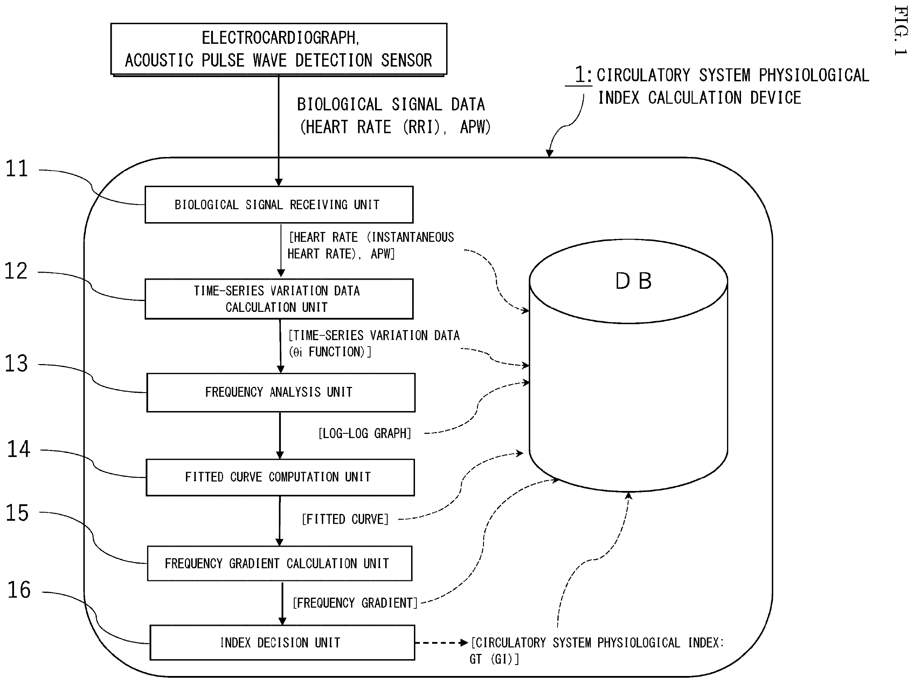

FIG. 1 illustrates a schematic configuration of an embodiment of a circulatory system physiological index calculation device 1. The circulatory system physiological index calculation device 1 of this embodiment is configured by a computer (the type of the computer is not limited and includes a personal computer, a microcomputer, and a portable information terminal such as a smartphone) and receives biological signal data and so on to execute predetermined processing.

Specifically, the circulatory system physiological index calculation device 1 has a storage unit (memory) in which a computer program is stored that causes the execution of procedures functioning as a biological signal receiving unit 11, a time-series variation data calculation unit 12, a frequency analysis unit 13, a fitted curve computation unit 14, a frequency gradient calculation unit 15, and an index decision unit 16. Note that examples of the storage unit (memory) include not only a recording medium such as a hard disk built in the circulatory system physiological index calculation device 1 configured by the computer, but also various removable recording media and a recording medium of another computer connected via communication means. The circulatory system physiological index calculation device 1 may also be implemented using an electronic circuit having a memory in which the abovementioned computer program is incorporated. That is, the biological signal receiving unit 11 may be configured as a computer's transmission/reception circuit, and the time-series variation data calculation unit 12, the frequency analysis unit 13, the fitted curve computation unit 14, the frequency gradient calculation unit 15, and the index decision unit 16 may be configured as a processing circuit that calculates time-series variation data from the biological signal data received by the transmission/reception circuit, frequency-analyzes it, finds fitted curves, calculates frequency gradients using the fitted curves, and finds one of the frequency gradients as an index correlating with blood pressure.

The computer program can also be provided by being stored in a recording medium. The recording medium storing the computer program may be non-transitory. The non-transitory recording medium is not limited, and examples thereof include recording media such as a flexible disk, a hard disk, CD-ROM, MO (magneto-optical disk), DVD-ROM, and a memory card. The computer program may also be transferred to the computer through communication lines to be installed.

The biological signal receiving unit 11 receives at least one piece of biological signal data out of heart rate and an acoustic pulse wave (APW) that is caused by left ventricular pressure variation and obtained from a body surface. The heart rate and the acoustic pulse wave are cardiocirculatory system physiological indexes, and their variations influence an effective blood volume to be blood pressure control factors. The heart rate can be an instantaneous heart rate calculated from an R-R interval (60/Δt/min: δt = RRI) of an electrocardiogram (ECG). In some cases, the instantaneous heart rate is calculated in an electrocardiograph, and the biological signal receiving unit 11 merely receives that data, or in some other cases, only the RRI is received by the biological signal receiving unit 11, and the instantaneous heart rate is calculated by the biological signal receiving unit 11. The biological signal receiving unit 11 of this embodiment may have either of these functions. Furthermore, for example, a wristwatch-type device, commonly referred to as a smartwatch, may also be used as the electrocardiograph. The acoustic pulse wave (APW) is sound/vibration information collected from the back, and an acoustic pulse wave detection sensor used in a drowsy driving warning device (Sleep Buster (registered trademark)) manufactured by Delta Tooling Co., Ltd. is used. Further, data (APW20) resulting from the filtering of the received data at 10 to 30 Hz is used as the acoustic pulse wave (APW).

The time-series variation data calculation unit 12 finds time-series data (time-series variation data) of variation (fluctuation) in the biological signal data received by the biological signal receiving unit 11. The time-series variation data is constructed as follows. Biological signal data in a predetermined period is compared with biological signal data in the subsequent predetermined period, and data representing variation between them are plotted. An atrial contraction-ventricular contraction phase, that is, an R-wave, obtained in ECG serves as a reference for the variation. In this embodiment, the variation is converted into angular information using the Lorenz plot method, and a function with the angle as its argument ("Δθi function") is found.

Specifically, the time-series variation data calculation unit 12 finds a reference gradient from a Lorenz plot in a reference time range, performs moving average processing on a relative gradient (characteristic amount of the variation), which is a difference between a gradient of each Lorenz plot created every predetermined time shorter than the reference time range and the reference gradient, and finds time-series data of the obtained relative gradient as the time-series variation data of the biological signal data.

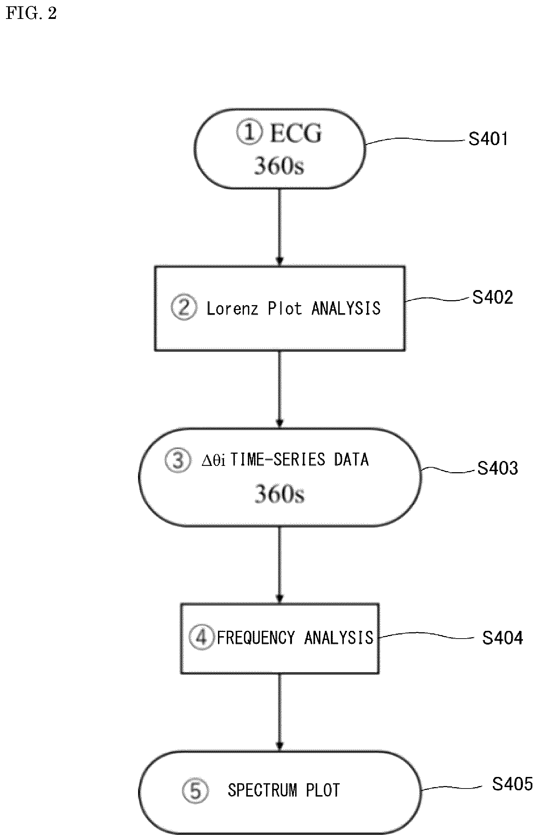

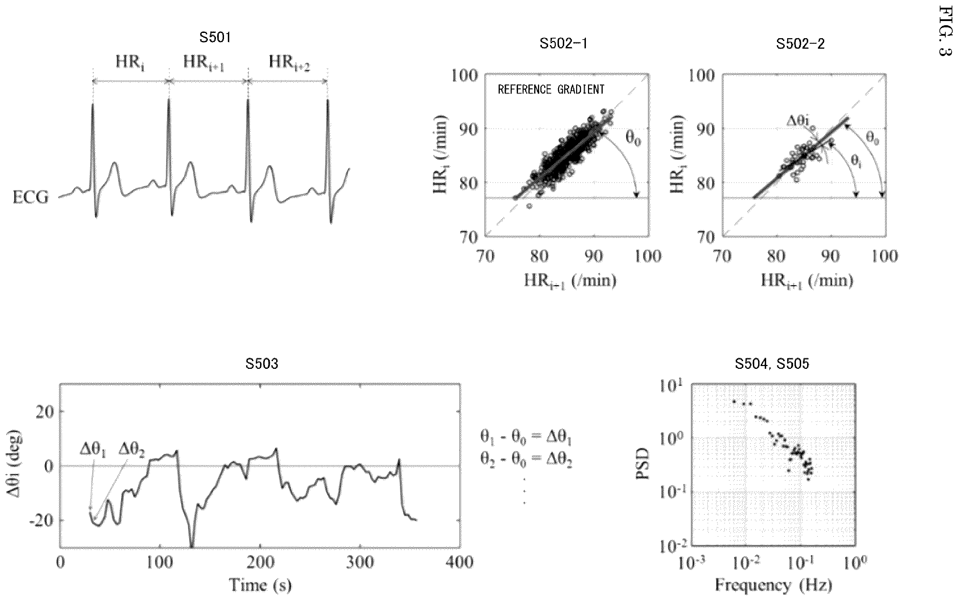

For example, as illustrated in FIG. 2 and FIG. 3, after instantaneous heart rates (HR) calculated from RRIs of an electrocardiogram are received (S401, S501), they are sequentially plotted with a value of the (i+1)-th heart rate taken on the horizontal axis and a value of the i-th heart rate (instantaneous heart rate) taken on the vertical axis, to create a Lorenz plot for the predetermined reference time range (for example, 360 seconds), and a gradient of a regression line (reference gradient) of a group of the plotted points is found (S402, S502-1). The reference time range is preferably 180 seconds or more, and more preferably 360 seconds. Time-series data of a physiological index contains information on variation amount, periodicity of the variation, and a manner/pattern of the variation.

A wave in a blood vessel is a superposition of waves generated by various factors. Although LF components are 0.04 to 0.15 Hz by definition, they are usually distributed in a frequency band of 0.06 to 0.11 Hz. On the other hand, very low frequency components (VLF components) defined as 0.003 to 0.04 Hz, which are long-term heart rate variability, contain information reflecting the regulation of effective blood volume by the circulatory control system and soundness of the entire living body. To capture a frequency component of 0.003 Hz, a measurement time of at least 1/0.003 = 333.4 seconds is required. By setting the measurement time to 360 seconds, it is possible to capture frequency components of 0.0028 Hz or higher.

For a time shorter than the reference time range (for example, in the case where the reference time range is 360 seconds, a measurement time shorter than that (for example, 30 seconds)), the Lorenz plot method is applied to plot values in a similar manner, a gradient of a regression line of a group of the plotted points in this short time window (short-time point group gradient) is found, and angular deviation between this short-time point group gradient (θi) and the reference gradient (θ0) is found as a relative gradient (Δθi) (S502-2 in FIG. 3). As described above, in this embodiment, a beat interval of the previous beat and that of the current beat are taken on the vertical axis and the horizontal axis respectively. If the Lorenz plot lies on a 45-degree straight line drawn from the origin on the graph, this situation indicates that variation per beat is zero. Therefore, the angular deviation (relative gradient (Δθi)) indicates information on instantaneous heart rate variability.

The short-time point group gradient (θi) is sequentially found for each time window by moving average processing. Since heart rate variability responds more quickly to stimulation of the parasympathetic nervous system than to that of the sympathetic nervous system, time resolution is decided in consideration of a heart rate regulation function and respiratory regulation by the parasympathetic regulation. In this embodiment, the time resolution is set to 3 seconds, and the time window for the moving average is set to 30 seconds. Then, the short-time point group gradient (θi) and the relative gradient (Δθi) are found for each time window, and time-series data of the relative gradient (Δθi) is created (S403 in FIG. 2, S503 in FIG. 3). This time series is time-series data indicating heart rate (instantaneous heart rate) variability (time-series variation data), that is, the abovementioned "Δθi function". Note that 30 seconds is twice as long as 15 seconds, which is an average time in which effects of activation/suppression of the sympathetic nerves are reflected.

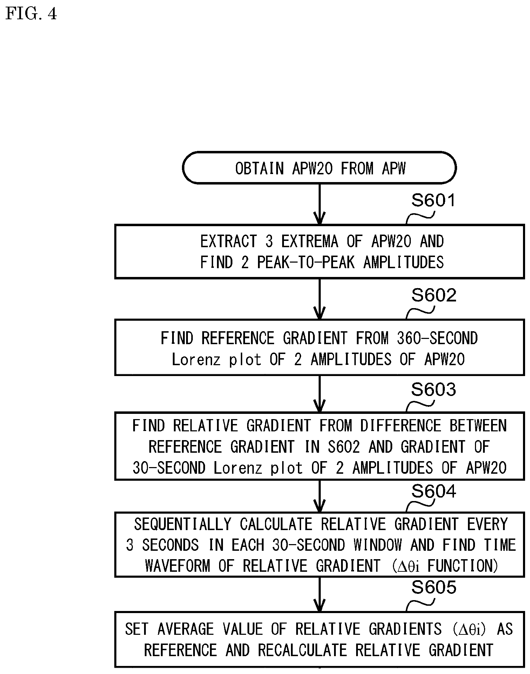

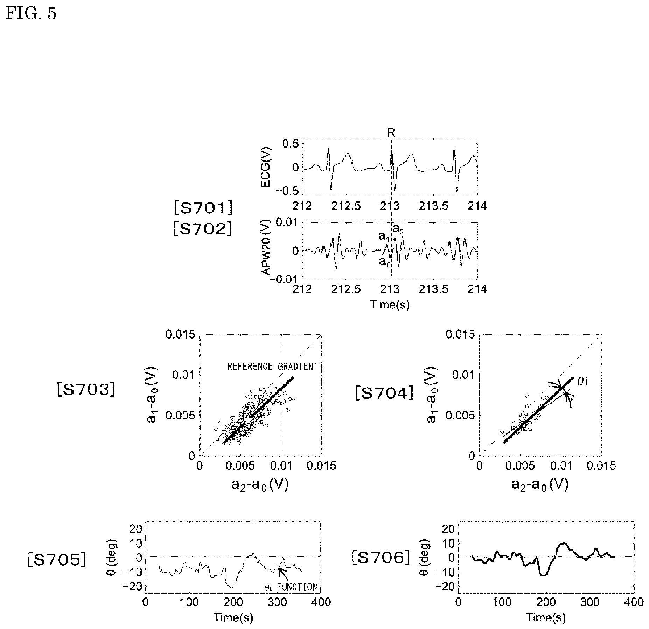

On the other hand, in the case where the biological signal data is APW20, three extrema (a0 to a2) in a period between atrial contraction and ventricular contraction are extracted from the received APW20 data, and two peak-to-peak amplitudes (a2-a0, a1-a0) are found (S601 to S602 in FIG. 4, S701 to S702 in FIG. 5). A Lorenz plot is created with these values on the horizontal axis and the vertical axis, a reference gradient (S603 in FIG. 4, S703 in FIG. 5) is found in a similar manner to the above, a relative gradient (S604 in FIG. 4, S704 in FIG. 5) is further found, and a Δθi function is created (S605 in FIG. 4, S705 in FIG. 5).

Note that the abovementioned VLF is one of the indexes of heart rate variability used for analyzing non-periodic components, and the analysis is performed in a frequency band of 0.003 to 0.04 Hz. However, since an irregular noise-like component leads to inaccurate correlation estimation, this noise component needs to be separated at the time of evaluation. Later-described "non-perturbation", which is a motion of baseline drift, does not have a specific frequency, and thus no peak is formed in the power spectrum. Therefore, it is necessary to eliminate influences of low-frequency variation components that last for a period of minutes or more, such as the renin-angiotensin system, out of eight arterial pressure control mechanisms. For this reason, the frequency analysis unit 13, which will be described next, applies a 0.006 Hz high-pass filter to the time-series data indicating heart rate (instantaneous heart rate) variability (time-series variation data), that is, the abovementioned "Δθi function", and applies a 0.08 Hz low-pass filter thereto to remove respiratory variation components in a frequency band of 0.1 Hz or higher, and uses the resultant data.

The frequency analysis unit 13 is a means for frequency-analyzing the Δθi function (to which the 0.006 Hz high-pass filter and the 0.08 Hz low-pass filter have been applied), which is the time-series data of the variation in the biological signal data and obtained from the time-series variation data calculation unit 12 (S404 in FIG. 2, S504 in FIG. 3), and displaying the result on log-log axes (S405 in FIG. 2, S505 in FIG. 3), and it captures the manner of variation in the biological signal data. S505 in FIG. 3 is an example where a power spectrum as the frequency analysis result is displayed on the log-log graph. This power spectrum on the log-log graph is displayed as dots respectively at many points where the power spectrum is calculated (power spectrum calculation points). Note that, in this specification, the display of the power spectrum displayed as these many dots is referred to as "plot display".

The fitted curve computation unit 14 performs an arithmetic operation to fit a fitted curve to the plot display on the log-log graph found by the frequency analysis unit 13. Specifically, the fitted curve computation unit 14 fits n-th degree polynomials or (n-1)-th degree polynomials to a plurality of types of analysis-target frequency bands set in the range from VLF to LF on the plot display of the power spectrum, and for the analysis-target frequency bands, a plurality of fitted curves represented by n-th or (n-1)-th degree polynomials with the smallest standard deviation of residuals with respect to the plots of the plot display of the power spectrum are found. It is preferable to use quartic and cubic polynomials obtained from the plot displays of power spectra on the log-log graphs resulting from the analyses regarding many subjects, thereby enabling substantially accurate analysis. However, if fitting fitted curves represented by higher-degree polynomials improves the accuracy of the circulatory system physiological index (GT or GI to be described later), higher-degree polynomials may also be employed.

The frequency bands where the fitted curves are fitted by the fitted curve computation unit 14 are preferably within a range, in the VLF to LF range, equal to or higher than a fundamental frequency that is determined based on a basic period, the basic period being the time required for the acute autoregulation of blood flow to return blood flow volume to a normal level when blood pressure rises. As a result, the later-described index obtained by the index decision unit 16 serves as a circulatory system physiological index that correlates with blood pressure and blood flow volume.

This is based on the following description on page 187 of "Guyton and Hall Textbook of Medical Physiology, Original Article, 13th Edition, published March 20, 2018, Author: John E. Hall, General Translation Supervisor: Yoshihiro Ishikawa et al., Publisher: Elsevier Japan KK". "In any tissue, an acute rise in arterial pressure causes an immediate rise in blood flow volume. However, within less than a minute, the blood flow volume in most tissues returns to the substantially normal range, even though the arterial pressure remains elevated. This phenomenon of blood flow volume returning to the normal level is called autoregulation. When autoregulation occurs, a relationship between local blood flow volume and arterial pressure in most body tissues becomes an "acute" curve represented by the solid line in Figure 17.5 (Figure shown on page 187 of this book)". With the blood flow volume taken on the vertical axis and the mean arterial pressure taken on the horizontal axis, this "acute" curve is a curve linearly changing when blood pressure is between approximately 100 mmHg and 150 mmHg, and changing nonlinearly when blood pressure is 100 mmHg or lower and when it is 150 mmHg or higher. Furthermore, since blood flow volume returns to the substantially normal range in less than a minute, the basic period of arterial pressure variability is 1/60 (seconds) ≈ 0.017 Hz. Further, according to Guyton's prior research (Role of the Baroreceptor Reflex in Daily Control of Arterial Blood Pressure and Other Variables in Dogs), the recovery time of the baroreceptor reflex is 21 seconds (1/21 = 0.0476 Hz).

From the above, it follows that the basic period of arterial pressure variability is 0.017 Hz when linearity is maintained, and in states of hypotension and hypertension, due to nonlinearity, there occur frequency components of 0.034 Hz, 0.051 Hz, 0.068 Hz, 0.085 Hz, 0.102 Hz, ... which are harmonic components of 0.017 Hz.

Meanwhile, LF components typically used in pulse wave measurement are commonly 0.04 to 0.15 Hz, but in the actual measurement results, they are mainly distributed between 0.06 Hz and 0.11 Hz. Therefore, to capture the influences of arterial pressure variability and baroreceptor reflex, the analysis-target frequency bands were studied primarily within a range up to 0.06 Hz, including 0.017 Hz, which is the basic period of arterial pressure variability, 0.034 Hz and 0.051 Hz, which are harmonic components of 0.017 Hz, and 0.0476 Hz of the baroreceptor reflex.

In a range lower than 0.017 Hz, it is assumed that non-periodic components are present. In a heart rate variability analysis, a frequency band where the non-periodic components appear is VLF, and VLF is defined as 0.003 to 004 Hz. Thus, between 0.017 and 0.06 Hz, the periodic components and the VLF-related non-periodic components coexist. The non-periodic components below 0.017 Hz exhibit a 1/f^{α} type fluctuation, which is what is known as baseline drift. Note that, in this specification, in some cases, this baseline drift is referred to as "non-perturbation", while short-term variation periodic motion appearing in both ±directions across the baseline is referred to as "perturbation". When the baseline drift is white noise with no frequency dependence, α = 0, and α = 1 in the case of so-called 1/f type fluctuation. When the amplitude of the baseline drift decreases in inverse proportion to a power of the frequency, α approaches 3. The case where α = 2 corresponds to signal noise generated by Brownian motion (Brownian noise).

The range from 0.017 to 0.06 Hz is a frequency band where the effects of the abovementioned autoregulation are exhibited, and is a region where the periodic components and the non-periodic components coexist. That is, it is a region where non-perturbation and perturbation, which are the baseline drift and the short-term variation periodic motion, coexist, and it has frequency characteristics having peaks superimposed on the 1/f^{α} type fluctuation.

Based on the above, in a normotensive individual in a resting state, the peaks of periodic components such as baroreceptor reflect are superimposed on a 1/f² type fluctuation of the non-periodic component in "non-perturbation", and a 1/f type fluctuation appears. In a hypertensive individual in a resting state, a 1/f type fluctuation becomes shallow and approaches a 1/f⁰ type fluctuation, and a characteristic extremum occurs in a frequency band around 0.0476 Hz or higher. In a hypotensive individual in a resting state, the power spectrum of the baroreceptor reflex decreases, resulting in a 1/f² type fluctuation.

Based on the above, the analysis-target frequency bands set by the fitted curve computation unit 14 are basically within the range of 0.017 to 0.06 Hz. However, since the occurrence of fluctuation varies among individuals, the bands can be adjusted within the range of 0.006 to 0.08 Hz, which is used when the time-series variation data calculation unit 12 finds the Δθi function.

While the fitted curve computation unit 14 basically sets fitted curves within the range of 0.017 to 0.06 Hz, it allows for adjustment to extend the analysis-target frequency bands to lower or higher frequencies. Therefore, the standard deviation of residuals is used as a criterion for setting the analysis-target frequency bands and the fitted curves more appropriately. Specifically, regarding a fitted curve set in the 0.017 to 0.06 Hz range, which is the basic analysis-target frequency band, and a fitted curve set in an analysis-target frequency band in a range from any frequency around 0.017 Hz (provided that it is 0.006 Hz or higher as described above) up to any frequency around 0.06 Hz (provided that it is 0.08 Hz or lower as described above), the fitted curve computation unit 14 finds residuals with respect to the plot display of the power spectrum obtained by the frequency analysis unit 13, and calculates the standard deviations thereof. Note that the frequency around 0.017 Hz is preferably adjusted to a power spectrum calculation point within a range whose lower limit is 0.006 Hz because, in this embodiment, the 0.006 Hz high-pass filter is set as described above, and whose upper limit is 0.034 Hz ,which is the first harmonic component of the basic period of arterial pressure variability, because data related to arterial pressure variability is indicated on a lower frequency side than 0.03 Hz as the boundary as will be described later, that is, within a 0.006 to 0.034 Hz range.

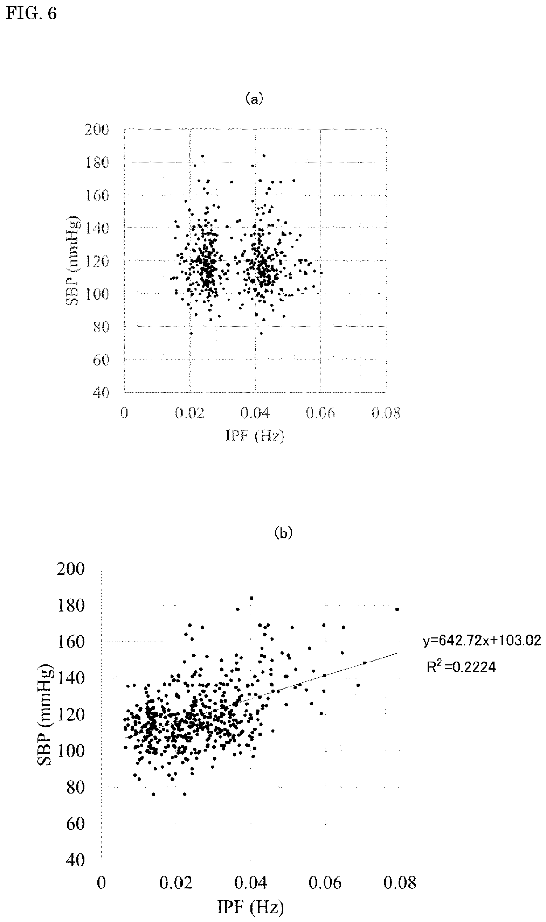

Here, a fitted curve represented by a quartic polynomial with the smallest squared sum of residuals was created in the 0.017 to 0.06 Hz range using data from a total of 171 subjects (260 cases), consisting of a healthy subject group (95 individuals) and a group of outpatients (76 individuals) with cardiovascular diseases, such as cardiac disease, hypertension, gout, diabetes, and dyslipidemia, or their risk factors (data of instantaneous heart rates calculated using RRIs obtained from ECG), and frequencies at inflection points (Inflection Point Frequency: IPF) were calculated, which are illustrated in the scatter plot in FIG. 6(a) The vertical axis represents systolic blood pressure and the horizontal axis represents IPF. In this scatter plot, between 0.017 and 0.06 Hz, the involvement of two control systems under nervous system governance is suggested. Clusters exist around 0.025 Hz and around 0.045 Hz with a boundary at 0.03 Hz. It is inferred that the former appeared in relation to arterial pressure variability because it is close to 0.017 Hz, while the latter appeared in relation to the baroreceptor reflex because it is close to 0.0476 Hz.

In general, when a linear component is mixed with white noise, a normal distribution results, and when a nonlinear component is mixed with white noise, a biased normal distribution results. Therefore, the analysis-target frequency band was expanded to a 0.006 to 0.08 Hz range, and a fitted curve reflecting both linear and nonlinear components was created. The fitted curve was represented by a quartic polynomial having a linear or nonlinear component in a frequency band where the standard deviation of residuals is the smallest and the residuals follow a normal distribution. In FIG. 6(b), the two IPFs of the fitted curve represented by the quartic polynomials are plotted, and it is seen from this plot that these two IPFs have a blood-pressure dependence, albeit with a weak correlation.

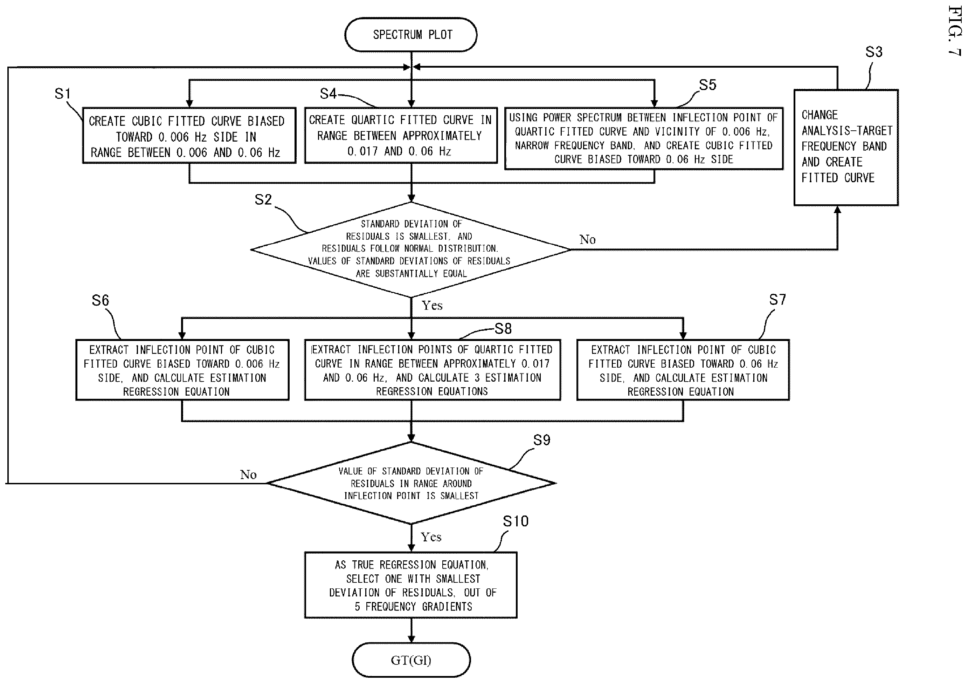

Approximate solutions can be calculated from gradient approximation and linear approximation regarding IPF. Gradient approximation characterizes a nonlinear component, while linear approximation characterizes a linear component. Accordingly, the inflection point-related frequency gradient (hereinafter referred to as GT or GI) serves as a blood pressure-dependent approximate solution regarding the inflection point of the fitted curve, and serves as a new index regarding blood pressure and blood flow volume (circulatory system physiological index). Based on these factors, the fitted curve computation unit 14 of this embodiment finds three cubic or quartic fitted curves (first to third fitted curves). This will be described based on the flowchart in FIG. 7.

### (1) First fitted curve (cubic polynomial fitted curve relevant to "non-perturbation")

This is a fitted curve represented by a cubic polynomial, with a 0.017 to 0.06 Hz range being set as a basic analysis-target frequency band.

The fitted curve computation unit 14 creates a fitted curve represented by a cubic polynomial in the 0.017 to 0.06 Hz range (S1). It then finds residuals between plots of the plot-displayed power spectrum and the fitted curve in the 0.017 to 0.06 Hz range, and determines whether a histogram of these residuals follows a normal distribution (S2).

If it does not follow the normal distribution ("No" in S2), the fitted curve computation unit 14 sets a new analysis-target band using a power spectrum in a range from 0.006 to the vicinity of 0.017 Hz (the upper limit is 0.034 Hz) (S3), and again finds a fitted curve represented by a cubic polynomial (S1). Thus, the first fitted curve becomes a cubic polynomial fitted curve biased toward the 0.006 Hz side, and has a high relevance to "non-perturbation".

The calculation is repeated, and if the fitted curves include one a histogram of whose residuals follows the normal distribution and whose standard deviation of residuals is the smallest, the determination in S2 is "Yes", and this cubic polynomial fitted curve is decided as the first fitted curve.

### (2) Second fitted curve (quartic polynomial fitted curve in which "non-perturbation" and "perturbation" coexist)

This is a fitted curve represented by a quartic polynomial, with the 0.017 to 0.06 Hz range being set as a basic analysis-target frequency band.

The fitted curve computation unit 14 creates a fitted curve represented by a quartic polynomial in the 0.017 to 0.06 Hz range (S4). It finds residuals between the plots of the plot-displayed power spectrum and the fitted curve in the 0.017 to 0.06 Hz range, and determines whether or not a histogram of these residuals follows the normal distribution (S2).

If it does not follow the normal distribution ("No" in S2), the fitted curve computation unit 14 reselects a power spectrum between both ends, namely, the vicinity of 0.017 Hz (within a range of 0.006 to 0.034 Hz) and the vicinity of 0.06 Hz (typically, a range from a value exceeding 0.034 to 0.06 Hz, but the maximum upper limit is 0.08 Hz), sets a new analysis-target band (S3), and again finds a fitted curve represented by a quartic polynomial (S4). Thus, the second fitted curve becomes a quartic polynomial fitted curve corresponding to an intermediate frequency band between 0.006 Hz and 0.06 Hz.

The calculation is repeated, and if the fitted curves include one a histogram of whose residuals follows the normal distribution and whose standard deviation of the residuals is the smallest, the determination in S2 is "Yes", and this quartic polynomial fitted curve is decided as the first fitted curve.

### (3) Third fitted curve (cubic polynomial fitted curve relevant to "perturbation")

This is a fitted curve represented by a cubic polynomial, with a power spectrum from the vicinity of a low frequency-side inflection point of the second fitted curve represented by the quartic polynomial to the vicinity of 0.06 Hz being set as an analysis-target frequency band.

The fitted curve computation unit 14 creates a fitted curve represented by a cubic polynomial in the range from the vicinity of the lower frequency-side inflection point of the second fitted curve to 0.06 Hz (S5). Then, in the analysis-target frequency band, it finds residuals between the plots of the plot-displayed power spectrum and the fitted curve. It then determines whether or not a histogram of these residuals follows the normal distribution (S2). If it does not follow the normal distribution ("No" in S2), it sets a new analysis-target band using the power spectrum in a range between the vicinity of the lower frequency-side inflection point of the second fitted curve and the vicinity of 0.06 Hz (S3), and again finds a fitted curve represented by a cubic polynomial (S5). Thus, the third fitted curve becomes a cubic polynomial fitted curve biased toward the 0.06 Hz side, and has a high relevance to "non-perturbation".

The calculation is repeated, and if the fitted curves include one a histogram of whose residuals follows the normal distribution and whose standard deviation of the residuals is the smallest, the determination in S2 is "Yes", and this cubic polynomial fitted curve is decided as the third fitted curve.

Note that the standard deviations of the residuals of the three fitted curves (1) to (3) are substantially equal values.

The frequency gradient calculation unit 15 calculates a frequency gradient for each of the fitted curves obtained by the fitted curve computation unit 14. In the case of the first and third fitted curves represented by the cubic polynomials, a tangent at an inflection point (an equation representing this straight line is referred to as an "estimated regression equation" in the drawing) is found, and its gradient is calculated as the frequency gradient (S6, S7 in FIG. 7). In the case of the second fitted curve represented by the quartic polynomial, tangents at two inflection points and a straight line connecting the two inflection points (equations representing these three lines are also referred to as "estimated regression equations" in the drawing) are found, and the gradients of these three lines are calculated as frequency gradients (S8 in FIG. 7). Note that, in this specification, the frequency gradient found as the gradient of the tangent at the inflection point is denoted as GT (Gradient of Tangent), and the frequency gradient found as the gradient of the straight line between the two inflection points is denoted as GI (Gradient of Inflections), regardless of the degree of the polynomial.

The index decision unit 16 decides a frequency gradient that is to be finally adopted as a circulatory system physiological index, among those of a total of five straight lines consisting of the four tangents at the inflection points and the one straight line between the inflection points, which are obtained by the frequency gradient calculation unit 15. Specifically, among the five straight lines (regression equations of the respective straight lines), one straight line is decided based on the standard deviation of residuals in the range around the inflection point (S9 in FIG. 7), and its frequency gradient is set as the final circulatory system physiological index (S10 in FIG. 7). The "range around the inflection point" refers to a predetermined power spectrum range centered at the inflection point, in the case of the tangents at the inflection points of the first fitted curve represented by the cubic polynomial, the second fitted curve, and the third fitted curve. Power spectra are found at appropriate frequency intervals, and the found power spectra are each plot-displayed as described above. In this embodiment, the standard deviation of the residuals of the tangent is found in a range of a plot display between ±2 points before and after the inflection point, which is a range relatively close to the inflection point. This is merely an example, and the range is not limited. The intervals at which the power spectra are found is also arbitrary. However, it is preferable to find the standard deviation of residuals in a range as close to the inflection point as possible, as in this embodiment. In terms of frequency unit, it is a range within ±0.03 Hz, preferably within ±0.02 Hz, and more preferably within ±0.01 Hz, centered at the inflection point.

In the case of the second fitted curve represented by the quartic polynomial approximation expression, the standard deviation of residuals is found not only in the ranges around the two inflection points but also in a predetermined plot display range, of the power spectrum, centered at the midpoint of the straight line connecting the two inflection points. That is, in the case of the second fitted curve, the standard deviations of residuals are found at three locations. Note that, in this specification, the "range around the midpoint", which is a predetermined power spectrum range centered at this midpoint, is also used as an element when one straight line is decided among the five straight lines, and therefore, for convenience, it will be included in the concept of the "range around the inflection point" in the explanation.

The index decision unit 16 compares the standard deviations of the residuals in the ranges around the five inflection points (including the range around the midpoint) of the five straight lines, and determines one with the smallest standard deviation of the residuals (S9 in FIG. 7). Then, the frequency gradient (GT or GI) of the one straight line with the smallest standard deviation of the residuals (the equation representing this straight line is referred to as a "true regression equation" in the drawing) is decided as the circulatory system physiological index (S 10 in FIG. 7).

However, if at least one of the standard deviations of the residuals in the ranges around the inflection points of the three frequency gradients (straight lines) of the second fitted curve represented by the quartic polynomial is smaller than the standard deviations of the residuals in the ranges around the inflection points of the frequency gradients (straight lines) of the first and third fitted curves represented by the cubic polynomials, it is preferable to consider the following determination:
Specifically, in this case, if the signs of the frequency gradients in the first and third fitted curves represented by the cubic polynomials match, the gradient GT with the matching sign is selected out of the gradients (GT) of the tangents at the two inflection points of the second fitted curve represented by the quartic polynomial. If the signs of the frequency gradients in the first and third fitted curves represented by the cubic polynomials do not match, the gradient between the inflection points (GI) of the second fitted curve represented by the quartic polynomial is selected. This allows for the easy selection of one frequency gradient from the three frequency gradients (straight lines) of the second fitted curve represented by the quartic polynomial. Furthermore, as is clear from the later-described experimental examples, in the case where this determination method is adopted, the standard deviation of residuals of the finally selected frequency gradient (straight line) is the smallest among the five in most cases.

Note that, in this specification, blood pressure is classified according to the classification defined in the "Guidelines for the Management of Hypertension 2019 (JSH2019)" (Published by: The Japanese Society of Hypertension), and systolic blood pressure is classified into "normal blood pressure: lower than 120 mmHg", "high-normal blood pressure: 120 to 129 mmHg", "elevated blood pressure: 130 to 139 mmHg", and "hypertension: 140 or higher (Grade I hypertension: 140 to 159; Grade II hypertension: 160 to 179; Grade III hypertension: 180 or higher)". Note that, in this specification, systolic blood pressure of 100 mmHg or lower is classified as "hypotension" in accordance with the WHO standard.

### Experimental Examples

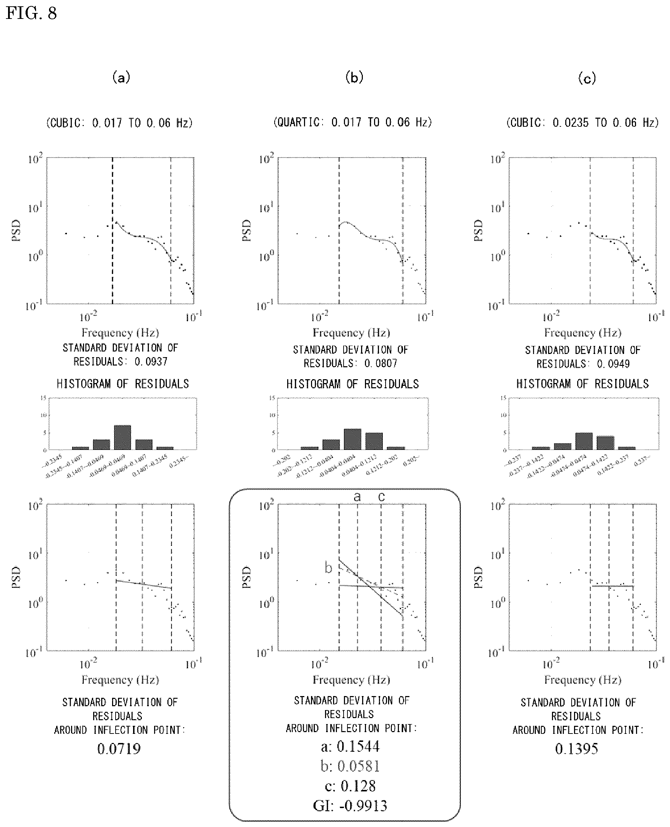

The results of analysis using the instantaneous heart rate data from the abovementioned 171 subjects (260 cases) mentioned are illustrated in FIGs. 8 to FIGs. 13. In (a), (b), and (c) in FIGs. 8 to FIGs. 13, the charts shown in the upper rows illustrate plot-displayed power spectra obtained by the time-series variation data calculation unit 12 finding Δθi functions, which are time-series variation data indicating variations in instantaneous heart rates, from data of the instantaneous heart rates received by the biological signal receiving unit 11, and by the frequency analysis unit 13 frequency-analyzing these.

The data from the 171 subjects were measured at the applicant's research facility and two hospitals. At the applicant's research facility, the subjects were each supported by an automobile seat during the measurement, and at the two hospitals, the subjects were each supported by a relaxation chair during the measurement. Experimental equipment, practitioners of the experiments, the subjects, analysis personnel, and the seasons in which the experiments were conducted are varied.

At the applicant's research facility, systolic blood pressure was measured twice using a brachial blood pressure monitor, and the average value was recorded. However, if there was a difference of 5 mmHg or more between the two measured values, systolic blood pressure was measured three to four times, and the average of the two closest measured values was recorded. The measurements at the hospitals were all performed only once.

Furthermore, from the 171 subjects (260 cases), the heart rate data were collected over a period of 360 seconds using an electrocardiograph while the subjects were in a resting seated position.

The solid curves illustrated in the upper rows of (a), (b), and (c) in FIGs. 8 to FIGs. 13 are fitted curves found by the fitted curve computation unit 14. Among these, one in each of the charts (a) is a first fitted curve represented by a cubic polynomial, one in each of the charts (b) is a second fitted curve represented by a quartic polynomial, and one in each of the charts (c) is a third fitted curve represented by a cubic equation that was found using a lower frequency-side inflection point of the second fitted curve. Furthermore, the charts in the upper rows illustrate only the first fitted curves, the second fitted curves, and the third fitted curves each with the smallest standard deviation of residuals among a plurality of fitted curves represented by cubic polynomials or quartic polynomials which are obtained in the varied analysis-target frequency bands as described above.

The charts in the middle rows illustrate histograms of residuals found by the fitted curve computation unit 14 regarding the first fitted curves, the second fitted curves, and the third fitted curves illustrated in the upper rows.

The charts in each of the lower rows illustrate a total of five straight lines consisting of tangents at four inflection points and one straight line between inflection points, which are calculated by the frequency gradient calculation unit 15, the standard deviations of the residuals in ranges around the inflection points (including ranges around the midpoints), which are calculated by the index decision unit 16, and the value of a frequency gradient (GT or GI) of the straight line with the smallest standard deviation of the residuals. Note that the standard deviation of the residuals is found at two power spectrum plots before and after each inflection point (including the midpoint). Furthermore, regarding the second fitted curve represented by the quartic polynomial in each of the charts (b), reference sign "a" indicates a lower frequency-side inflection point, and reference sign "c" indicates the frequency of a higher frequency-side inflection point. Reference sign "b" indicates the straight line itself connecting the two inflection points, while the numerical value on the right of "b:" at the bottom of the graph represents the standard deviation of residuals in the range around the midpoint between the inflection points "a" and "c".

From the comparison of the standard deviations of the residuals shown in the lower row in FIGs. 8(a) to (c), it is seen that the smallest value is 0.0581, which is the value in the range around the midpoint of the straight line "b" between the inflection points of the second fitted curve. Therefore, the index decision unit 16 decides the frequency gradient: GI = -0.09913 of the straight line "b" between the inflection points, as the circulatory system physiological index.

Note that the sign of the gradient of the first fitted curve biased toward the 0.006 Hz side is negative, and the sign of the gradient of the third fitted curve biased toward the 0.06 Hz side is positive or substantially zero. Accordingly, since the signs of the two gradients do not match, this result is the same as a determination to select GI in the second fitted curve.

GI possesses two characteristics: the frequency gradient in the vicinity of 0.017 Hz and that in the frequency band of 0.03 to 0.06 Hz.

This subject is a normotensive subject whose systolic blood pressure, as measured by the brachial blood pressure monitor, is 116 mmHg (heart rate at the time of measurement: 60/min).

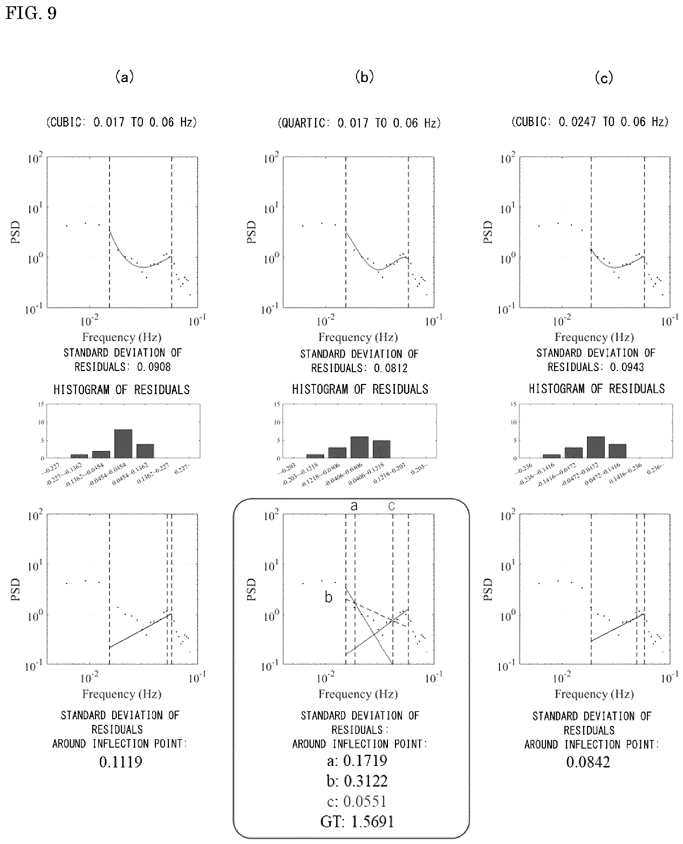

From the comparison of the standard deviations of the residuals shown in the lower row in FIGs. 9(a) to 9(c), it is seen that the smallest value is 0.0551, which is the value in the range around the inflection point "c" of the second fitted curve. Since the inflection point "c" is the higher frequency-side inflection point, the index decision unit 16 decides the frequency gradient: GT = 1.5691 of the tangent at the inflection point "c", as the circulatory system physiological index.

Note that the signs of the gradient of the first fitted curve biased toward the 0.006 Hz side and the gradient of the third fitted curve biased toward the 0.06 Hz side are both positive, and thus the signs of the gradients match. Accordingly, this result is the same as a determination to select GT at the inflection point "c" having the positive gradient in the second fitted curve.

This subject is a Grade **III** hypertensive subject whose systolic blood pressure, as measured by the brachial blood pressure monitor, is 184 mmHg (heart rate at the time of measurement: 108/min).

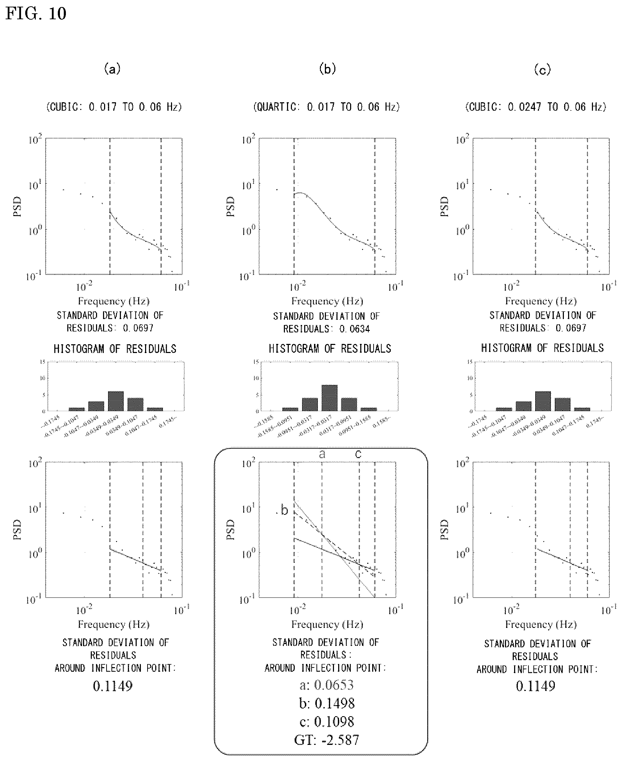

From the comparison of the standard deviations of the residuals shown in the lower row in FIGs. 10(a) to (c), it is seen that the smallest value is 0.0653, which is the value in the range around the inflection point "a" of the second fitted curve. Since the inflection point "a" is the lower frequency-side inflection point, the index decision unit 16 decides the frequency gradient: GT = -2.587 of the tangent at the inflection point "a", as the circulatory system physiological index.

Note that the signs of the gradient of the first fitted curve biased toward the 0.006 Hz side and the gradient of the third fitted curve biased toward the 0.06 Hz side are both negative, and thus the signs of the gradients match. In addition, the gradients of all the three straight lines in the second fitted curve are also negative. In this case, GT of the tangent at the inflection point "a" where the standard deviation of the residuals is smallest in the second fitted curve is adopted.

This subject is a hypotensive subject whose systolic blood pressure, as measured by the brachial blood pressure monitor, is 76 mmHg (heart rate at the time of measurement: 108/min).

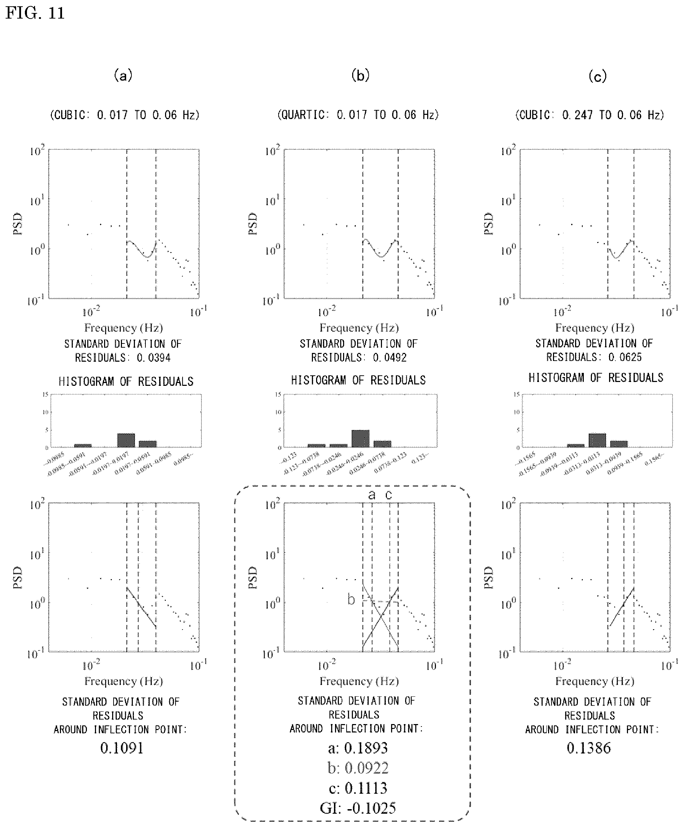

From the comparison of the standard deviations of the residuals shown in the lower row in FIGs. 11(a) to (c), it is seen that the smallest value is 0.0922, which is the value in the range around the midpoint of the straight line "b" between the inflection points of the second fitted curve. Therefore, the index decision unit 16 decides the frequency gradient: GI = -0.1025 of the straight line "b" between the inflection points, as the circulatory system physiological index.

Note that the sign of the gradient of the first fitted curve biased toward the 0.006 Hz side is negative, and the sign of the gradient of the third fitted curve biased toward the 0.06 Hz side is positive. Accordingly, since the signs of the two gradients do not match, this result is the same as a determination to select GI in the second fitted curve.

This subject is a high-normal blood pressure subject whose systolic blood pressure, as measured by the brachial blood pressure monitor, is 137 mmHg (heart rate at the time of measurement: 58/min).

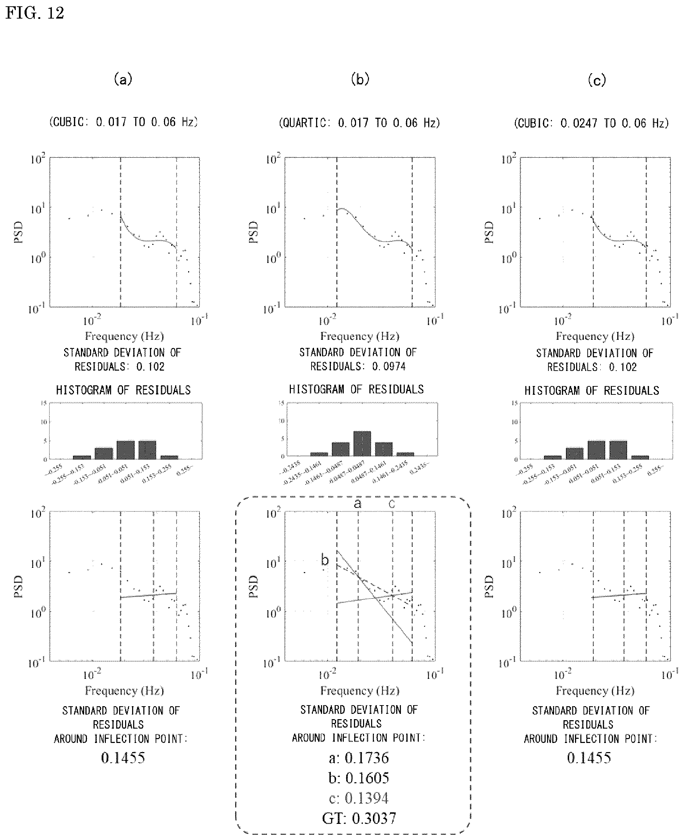

From the comparison of the standard deviations of the residuals shown in the lower row in FIGs. 12(a) to (c), it is seen that the smallest value is 0.1394, which is the value in the range around the inflection point "c" of the second fitted curve. Since the inflection point "c" is the higher frequency-side inflection point, the index decision unit 16 decides the frequency gradient: GT = 0.3037 of the tangent at the inflection point "c", as the circulatory system physiological index.

Note that the signs of the gradient of the first fitted curve biased toward the 0.006 Hz side and the gradient of the third fitted curve biased toward the 0.06 Hz side are both positive, and thus the signs of the gradients match. Therefore, this result is the same as a determination to select GT with the positive gradient at the inflection point "c" in the second fitted curve.

This subject is a Grade I hypertensive subject whose systolic blood pressure, as measured by the brachial blood pressure monitor, is 149 mmHg (heart rate at the time of measurement: 74/min).

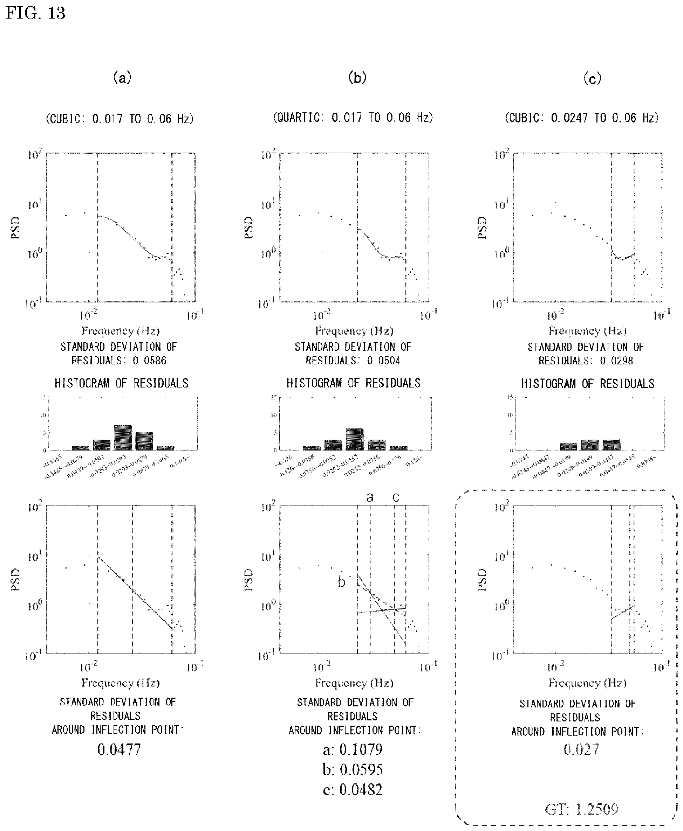

From the comparison of the standard deviations of the residuals shown in the lower row in FIGs. 13(a) to (c), it is seen that the smallest value is 0.027, which is the value in the range around the inflection point of the third fitted curve. Therefore, the index decision unit 16 decides the frequency gradient: GT = 1.2509 of the tangent at this inflection point, as the circulatory system physiological index.

This subject is a Grade II hypertensive subject whose systolic blood pressure, as measured by the brachial blood pressure monitor, is 168 mmHg (heart rate at the time of measurement: 83/min).

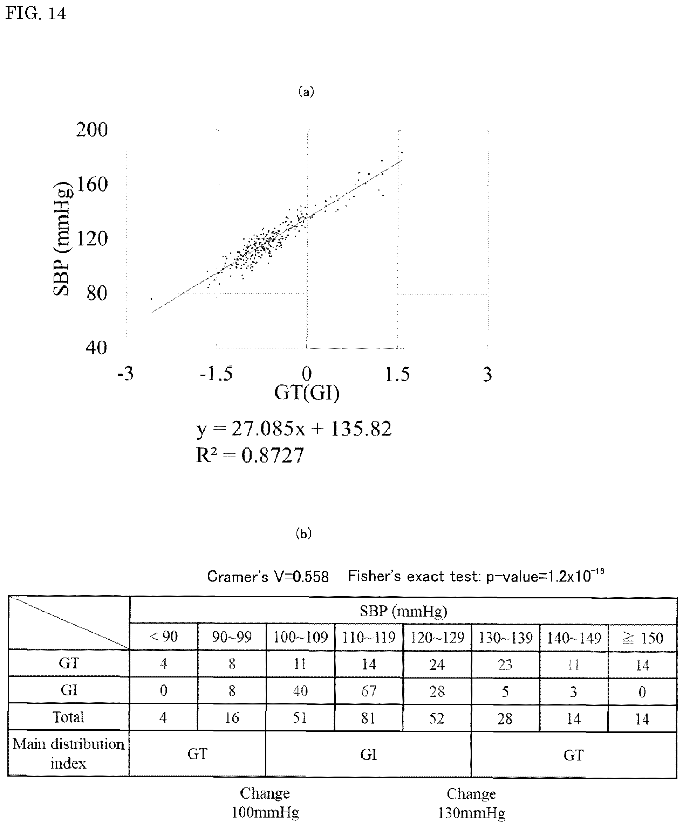

FIG. 14(a) is a chart illustrating a correlation between the values of the frequency gradients (GT (GI)) found in the above-described manner and the values of the systolic blood pressures (SBP) measured using the brachial blood pressure monitor, regarding all the 171 subjects (260 cases). A blood pressure estimation formula represented by a regression equation was y = 27.085x + 135.82, and a coefficient of determination was R² = 0.8727, showing a high correlation.

In the Guyton and Hall Textbook of Medical Physiology cited above, following an explanation that autoregulation occurs and a relationship between local blood flow volume and arterial pressure in many body tissues is represented by the abovementioned "acute curve", there is a description regarding the "acute curve" (hereinafter, in this specification, the characteristics of this curve are referred to as "acute curve characteristics"), as follows. "Please note that, when arterial pressure is between approximately 70 and 175 mmHg, blood flow volume increases only by 20 to 30% even though the arterial pressure increases by 150%. In some tissues, such as the brain and the heart, this autoregulation is even more precise". By combining these acute curve characteristics with the regression equation shown in FIG. 14(a) and eliminating blood pressure, it is possible to rephrase the above explanation as: "When arterial pressure is between approximately 70 and 175 mmHg, blood flow is regulated within a frequency gradient (GT or GI) range of -3 to 1.5, during which blood flow volume increases by only 20 to 30%". In other words, the regression equation in FIG. 14(a) is considered to have been obtained as a result of the association of autoregulation having the acute curve characteristics with blood pressure regulation. From this perspective as well, it can be said that the frequency gradient (GT or GI) found in this embodiment is suitable as a circulatory system physiological index related to the functions of regulating blood flow volume and blood pressure.

Furthermore, FIG. 14(b) is a cross-tabulation table showing a relationship of the measured systolic blood pressures (SBP) with GT and GI. The presence or absence of an association was determined using Fisher's exact test, the level of the association was analyzed using Cramer's coefficient of association, and a test of independence between the frequency gradient (GT or GI) and the systolic blood pressure (SBP) was conducted.

The calculated Cramer's coefficient of association was 0.558, showing that there is a strong correlation between the frequency gradient (GT or GI) and the systolic blood pressure (SBP). Note that Cramer's coefficient of association is the χ² value normalized to 0 to 1 and is an index representing the strength of association. As for the index indicating the strength of the association between the items in the row and column of the cross-tabulation table, it is defined that 0.1 to 0.25 indicate weak association, 0.25 to 0.5 indicate a moderate association, and values exceeding 0.5 indicate a strong association.

Further, as illustrated in FIG. 14(a), the y-intercept of the regression equation is 135.82 mmHg, and the cross-tabulation table also shows that the frequency gradient switches from GI to GT when SBP becomes 130 to 139 mmHg. When SBP exceeds 130 mmHg and the nervous system's dominance over the arterial pressure control mechanism increases, the power spectrum in a 0.03 to 0.05 Hz frequency band increases, which is reflected on the fitted curve. This increase occurs due to the mixing of a nonlinear component into the fitted curve, and the approximate solution to the nonlinear component becomes positive GT. On the other hand, when the increase in the nervous system dominance remains within a linear region, the linear approximate solution becomes GI. When blood pressure falls below 100 mmHg, a nonlinear component is mixed into the low-frequency band in a biased manner, and the approximate solution to the nonlinear component becomes a negative GT.

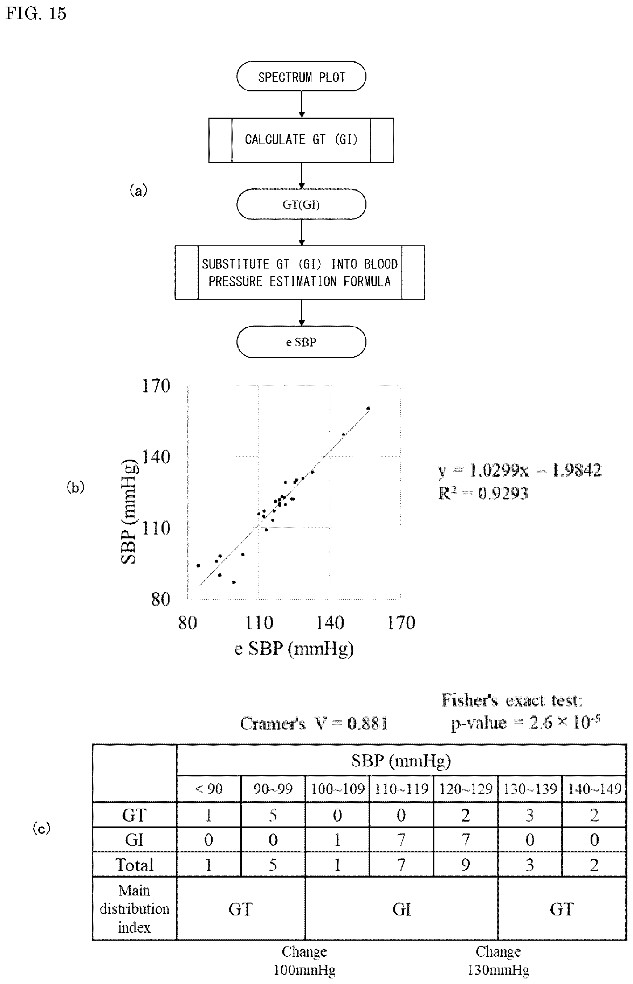

As illustrated in the flowchart in FIG. 15(a), by substituting the frequency gradient (GT or GI), which is the circulatory system physiological index obtained by the index decision unit 16, into the abovementioned blood pressure estimation formula, it is possible to find the estimated systolic blood pressure (eSBP). When the values of GT or GI found in FIGs. 8 to FIGs. 13 were substituted into the blood pressure estimation formula, the estimated blood pressure values were 109 mmHg, 178 mmHg, 66 mmHg, 133 mmHg, 144 mmHg, and 170 mmHg, respectively, and in all the cases, values close to the measured blood pressure values were obtained.

FIG. 15(b) is a chart illustrating a correlation between estimated systolic blood pressures (eSBP) and systolic blood pressures (SBP). The estimated systolic blood pressures are obtained by finding frequency gradients (GT or GI) regarding 28 subjects (healthy individuals) different from the abovementioned 171 subjects and substituting the frequency gradients into the blood pressure estimation formula, and the systolic blood pressures are simultaneously measured from these subjects using a brachial blood pressure monitor. As shown in FIG. 15(b), the coefficient of determination was R² = 0.9293, and in the cross-tabulation table in FIG. 15(c), Cramer's coefficient of association was 0.881, showing an extremely high correlation.

The 28 subjects were male subjects in their 30s to 80s who were not outpatients with cardiovascular diseases. The measurement postures were seated and recumbent (lateral or supine), and data for 360 seconds measured by an electrocardiograph were used.

The postures of the 171 subjects during the ECG measurements were a resting seated position, whereas the measurement postures of these 28 subjects were seated and recumbent (lateral or supine). Although the measurements were conducted in various postures in this manner, the coefficient of determination for estimation accuracy exceeds 0.9, as described above. Meanwhile, the measured values of systolic blood pressures used to find the blood pressure estimation formula were all data measured in a seated position. Therefore, by using the frequency gradient (GT or GI) of this embodiment, it is possible to estimate blood pressure without limiting the measurement posture, and it possesses robustness that is less susceptible to the measurement environment and provides high utility even for blood pressure measurement in states other than a resting state.

In the cross-tabulation table in FIG. 15(c), when the measured systolic blood pressure is lower than 100 mmHg and when it is 130 mmHg or higher, the frequency gradient is GT. When it is in a range of 100 mmHg or higher and lower than 130 mmHg, the frequency gradient is GI. 100 mmHg and 130 mmHg are the boundary blood pressures at which the frequency gradient changes between GT and GI.

### (Evaluation of Biological State Using Circulatory System Physiological Index)

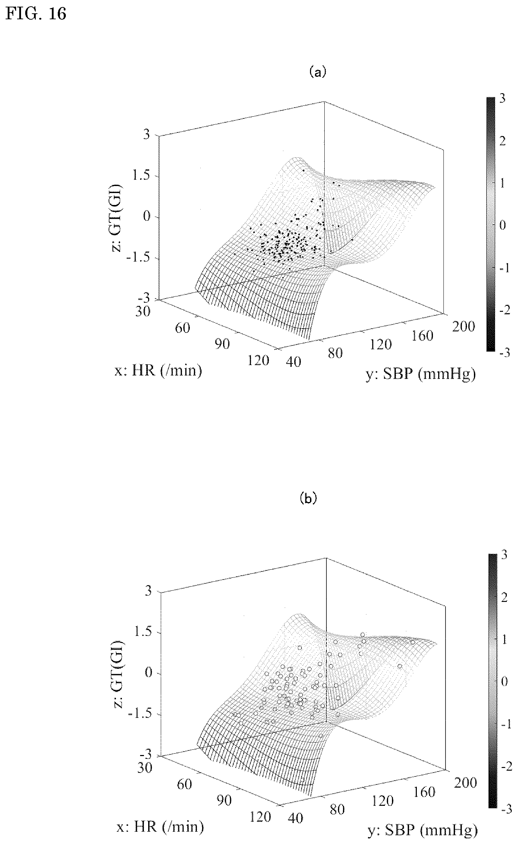

Heart rate and blood pressure increase when an exercise load is applied, and then gradually return to their original levels when the exercise load is removed and a resting state is entered. An index of blood flow control based on these heart rate and blood pressure is the frequency gradient (GT or GI) found in this embodiment. Therefore, as illustrated in FIGs. 16(a), (b), three-dimensional response surfaces were created using the data from the abovementioned 171 subjects (260 cases), with heart rate and blood pressure taken on the two horizontal axes and the frequency gradient (GT or GI (denoted as "GT (GI)" in the drawings)) taken on the vertical axis. Specifically, these are three-dimensional response surfaces in each of which the heart rate (HR) and the systolic blood pressure (measured value: SBP) are used as explanatory variables, and the frequency gradient (GT (GI)) is used as an objective variable. Note that FIG. 16(a) is a three-dimensional response surface created using the data from the healthy subject group (95 individuals), and FIG. 16(b) is a three-dimensional response surface created using the data from the group of outpatients with heart disease and risk factors (76 individuals).

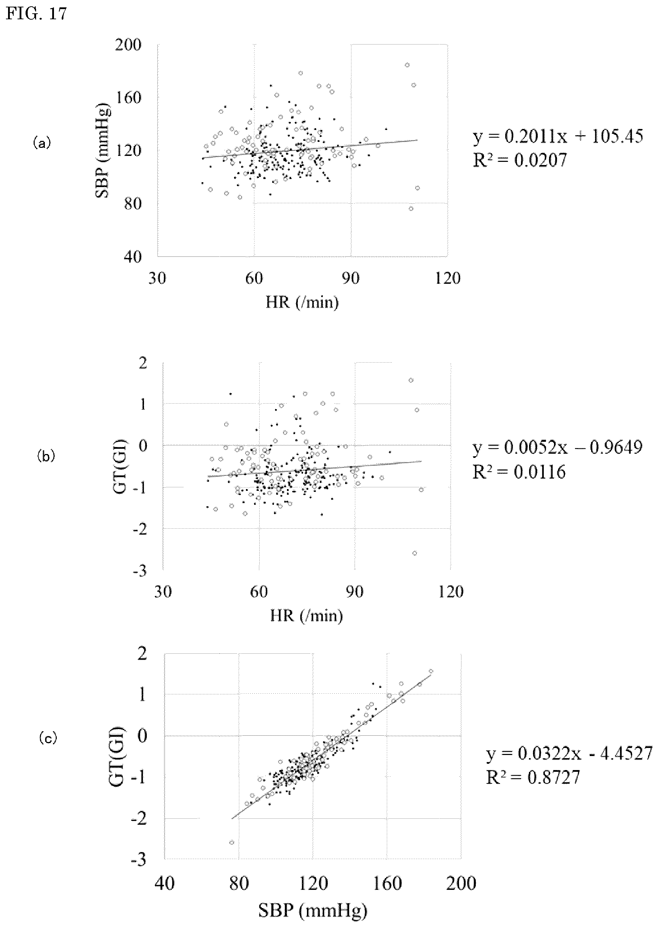

FIGs. 17(a) to (c) illustrate cross-sections of the three-dimensional response surface. The GT (GI)-SBP cross-sectional view in FIG. 17(c) shows a strong correlation, whereas neither of the SBP-HR scatter plot in FIG. 17(a) and the GT (GI)-HR scatter plot in FIG. 17(b) shows any correlation. With respect to the heart rate axis, these plots represented a substantially reflection transformation.

The three-dimensional response surfaces illustrated in FIGs. 16(a), (b) are represented by quartic polynomials. The data from the healthy subject group (95 individuals) are distributed in the central portion approximately represented by a straight line, whereas the data from the outpatient group with heart disease and risk factors (76 individuals) are widely distributed up to the edge of the surface represented by a fitted curve, and from this, it has been found that these can be used to identify abnormal values related to blood pressure and heart rate.

For example, in the case of the healthy subject group in a high blood pressure state, the surface exhibits nonlinearity, and the heart rate varies at 90/min or less. In a low blood pressure state, the surface exhibits linearity, and the heart rate varies at 60/min or more.

On the other hand, in the case of the outpatient group in a high blood pressure state, the surface exhibits nonlinearity, the heart rate varies at 90/min or more, and cases were observed where the heart rate scattered at the four corners of the surface represented by the quartic polynomial. In a low blood pressure state, the heart rate varies at 60/min or less, and cases were observed where it scattered on the surface exhibiting nonlinearity, and the value of the frequency gradient (GT or GI) varies significantly when blood pressure or heart rate varies. These cases where it scatters on the nonlinear surface indicate the possibility of impairment in the function of the autonomic nervous system, suggesting that the biological system is approaching a state of lost harmony due abnormality or dysfunction of organs/internal organs. Humans have a function of the endocrine system control including the renin-angiotensin-aldosterone system (RAAS), which is a regulatory function concurrent with the nervous system control, and by using the frequency gradient (GT or GI), it is possible to estimate the process in which both the nervous system control and the endocrine system control functions decline.

### (Verification Using Data from Same Individuals)

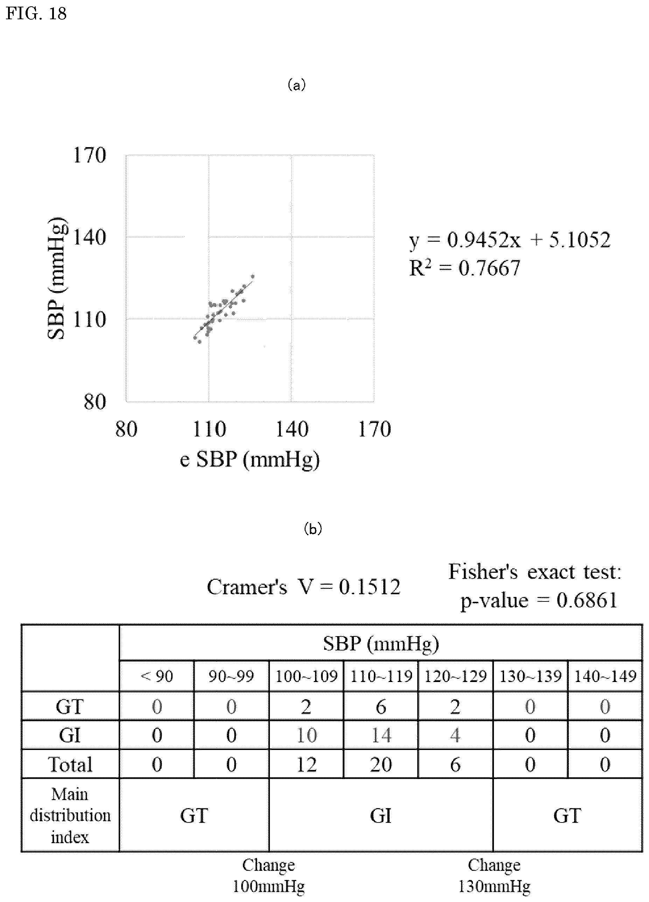

FIGs. 18 to FIGs. 20 are the results of experiments conducted on three males aged 34 (healthy individual), aged 55 (outpatient: undergoing medication for hypertension), and aged 67 (outpatient: undergoing medication for hypertension), in which verification was performed on values of their estimated systolic blood pressures (eSBP), which are found by the abovementioned blood pressure estimation formula using instantaneous heart rate data in the past one year, and measured values of their brachial systolic blood pressures taken at the time of the measurement of the instantaneous heart rates. All the cases showed high coefficients of determination, leading to the confirmation that the blood pressure estimation formula derived from the data of the 171 individuals (260 cases) is also effective for estimating the blood pressure values of the same individuals.

Further, in the case of the 34-year-old male in FIGs. 18, the measured value of systolic blood pressure varied within the normal blood pressure range of 100 to 129 mmHg. Cramer's coefficient of association showed a weak correlation, and there was no independence between the items of GI/GT and SBP. Fisher's exact test also showed no significant difference between the variation in the measured values of systolic blood pressure and GI or GT. An individual who can maintain normal blood pressure in this state is recognized as having favorable nervous system control.

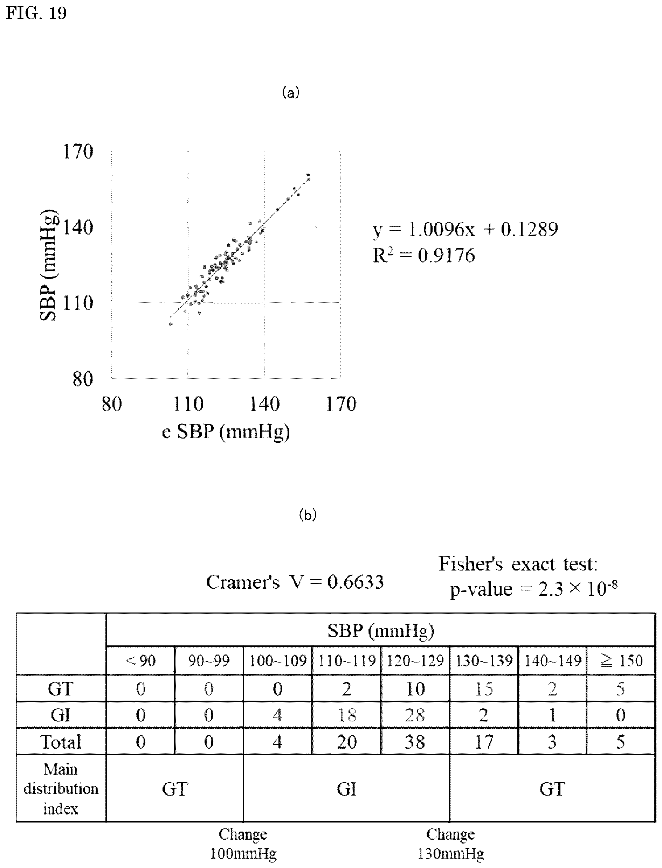

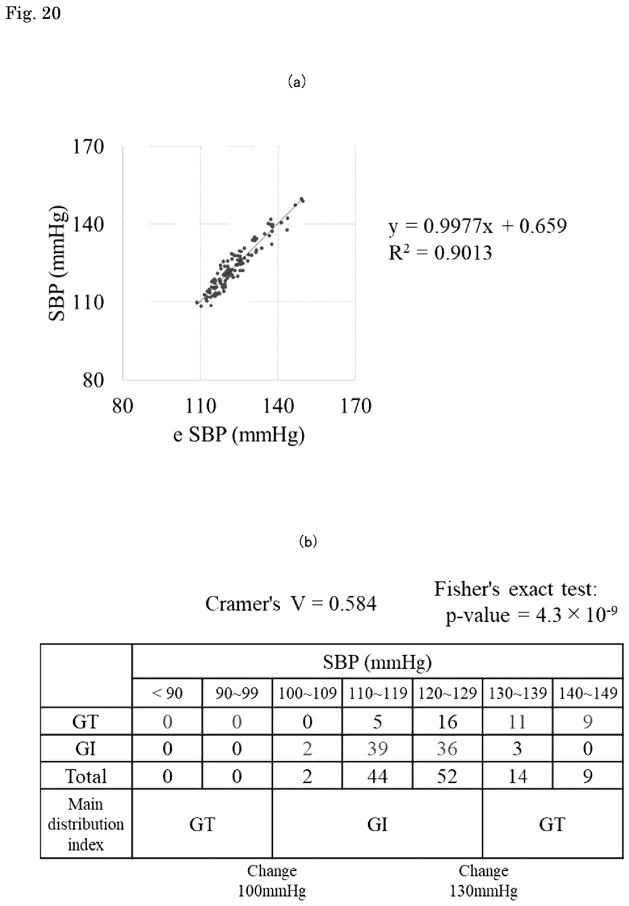

On the other hand, in the cases of the 55-year-old male subject and the 67-year-old male subject in FIGs. 19 and FIGs. 20, variation amounts of the measured values of systolic blood pressures were 104 to 164 mmHg and 108 to 150 mmHg, respectively. Both Fisher's exact test and Cramer's coefficient of association indicated a correlation. The independence of GT and GI could be verified from the coefficient of association, and 100 mmHg and 130 mmHg were the boundaries where the frequency gradient switched from GT to GI and from GI to GT. It is recognized that GT indicates the possibility of blood pressure control in which the effects of aging and medication are reflected as an integrated effect. GI indicates a state in which blood pressure control is performed by nervous system dominance, and it is considered that the effects of aging and medication are small in this state.

### (Application of Frequency Gradient (GT (GI)))

The homeostasis of cardiac output is maintained by the homeostasis of heart rate and blood pressure. Therefore, the fact that the homeostasis of heart rate and blood pressure is defined by GT (GI) means that the homeostasis of cardiac output is defined by GT (GI). If variations in heart rate and blood pressure can be captured by a control system related to cardiac function, it is possible, for example, to calculate GT (GI) originating from the Frank-Starling mechanism that constitutes cardiac function, enabling the extraction of a new clinically significant index related to hemodynamics.

In the above embodiment, systolic blood pressure was estimated from instantaneous heart rate measured from an electrocardiogram, but in principle, it is impossible to estimate diastolic blood pressure. Here, to calculate GT (GI) originating from the Frank-Starling mechanism, an approximation expression regarding the shape and propagation velocity of a solitary wave is used, it is developed into an expression representing a left ventricular pressure waveform to create the left ventricular pressure waveform, and a comparative study with an acoustic pulse wave (APW), which is a measured waveform, is performed, whereby parameters for diastolic blood pressure estimation can be extracted. Note that, as described in the above embodiment, systolic blood pressure can be estimated using APW.

A left ventricular pressure waveform generated during atrial contraction in the ventricular filling phase was created by a solution based on the series expansion of complex potentials, using an approximation expression by which a two-dimensional wave generated in an x-y plane was found in accordance with Rayleigh. Next, a left ventricular pressure waveform exhibiting the maximum rate of internal pressure rise during the isovolumetric contraction phase was created, and a left ventricular pressure waveform during the ventricular ejection phase after the opening of the aortic valve was created, which was then normalized by systolic blood pressure. Subsequently, a left ventricular pressure waveform in the time phase from the closing of the aortic valve to the opening of the mitral valve during the isovolumetric relaxation phase was created, and an attempt was made to create an apex beat wave (CAB). Note that the approximation expression η(x, t) used to create the left ventricular pressure waveform in the ventricular cycle is expressed by the following expression:
[Expression 1] $η \left(x, t\right) = a sec {h}^{2} \left\{\sqrt{\frac{3 ε}{4 {h}^{2} \left(1+ε\right)}}\left(x-ct\right)\right\} = a sec {h}^{2} \left\{\frac{g}{2 {c}^{2}}\sqrt{3 ε \left(1+ε\right)}\left(x-ct\right)\right\}$

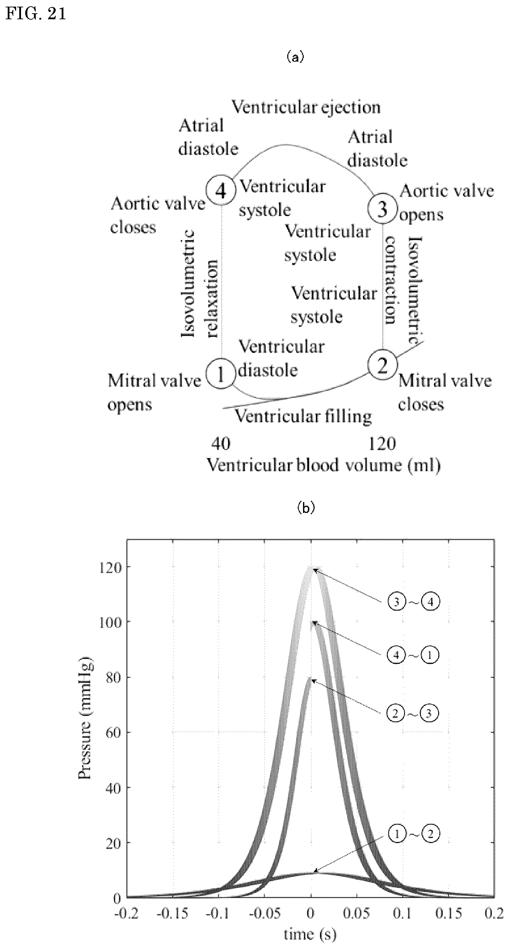

ε is an ejection fraction, and "h" is an index (m) that is the height of potential energy replacing the mechanical energy of blood flow. "a" is the left ventricular pressure (mmHg) and "c" is the flow velocity of blood (m/sec). Using the information shown in Table 1 when heart rate was 60/min, a simulation of the left ventricular pressure waveform for each ventricular cycle (Pseudo left ventricular pressure (analysis value: p-LVP)) shown in FIG. 21(a) was created. Data interpolation was performed within the duration time to create p-LVP fitted curves (FIG. 21(b)).

**[Table 1]**

| | Duration time (s) : z-axis | c (m/s) | h₁ (m) | ε | Pressure (mmHg) |
|---|---|---|---|---|---|
| ①~② | 0.466 | 0.06 | 0.175 × 10⁻³ | 1.1 | 9 |
| ②~③ | 0.05 | 0.205 | 1.6 × 10⁻³ | 1.67 | 80 |
| ③~④ | 0.404 | 0.13 | 0.65 × 10⁻³ | 1.67 | 120 |
| ④~① | 0.08 | 0.16 | 1.2 × 10⁻³ | 1.1 | 100 |

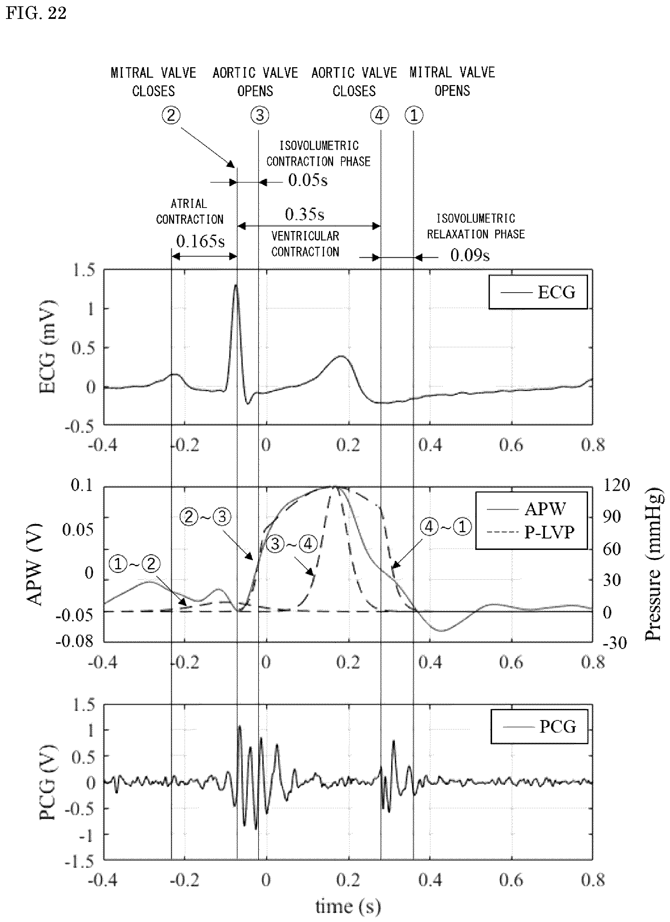

FIG. 22 illustrates measured ECG, APW, and phonocardiogram (PCG), where the p-LVP fitted curve and p-LVP are represented by the broken lines. dP/dtₘₐₓ of the p-LVP fitted curve, which is normalized by systolic blood pressure, during the isovolumetric contraction phase substantially matches the maximum rate of internal pressure increase (dP/dtₘₐₓ) of APW. This suggests the usefulness of the approximation expression for the pressure waveform η(x, t). Note that APW shown here is an apex beat wave (CAB) measured from the apex of the heart (refer to Patent Documents 3 and 4), and that with a heart rate of 58/min was used.

### (Cardiovascular Model of Double-Well Potential System Using Fitted Curves Represented by n-th Degree Polynomials)

Non-periodic components are related to the Frank-Starling mechanism and RAAS, while periodic components are related to various neural regulatory factors. The baroreceptor feedback mechanism and the central nervous ischemic mechanism operate in seconds or minutes. Through the collaborative action of these neural mechanisms, in response to a drop in arterial pressure, the chemoreceptor mechanism normalizes arterial pressure by increasing venous return, increasing heart rate and cardiac contractility, and obstructing arterial blood flow through the constriction of peripheral arterioles. In response to a sudden rise in arterial pressure, it works in the opposite direction to lower the pressure in the normal direction. This constitutes the abovementioned periodic component.

Those operating in minutes or hours include the renin-angiotensin system which raises arterial pressure, the stress relaxation system which lowers arterial pressure to recover the normal value, and the capillary fluid shift mechanism which is a mechanism for lowering or raising the pressure by decreasing or increasing the blood flow volume in the circulatory system. These constitute the abovementioned non-periodic components. Arterial pressure is kept stable over a long period through the arterial pressure control by the interaction between the RAAS and the renal-body fluid mechanism. This corresponds to a non-periodic component that creates individual differences.

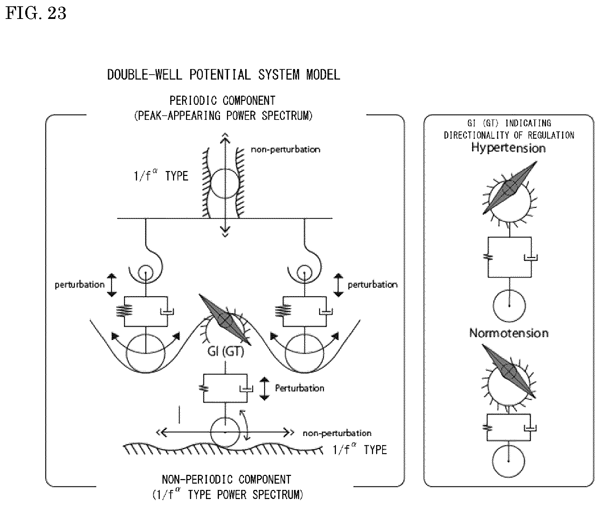

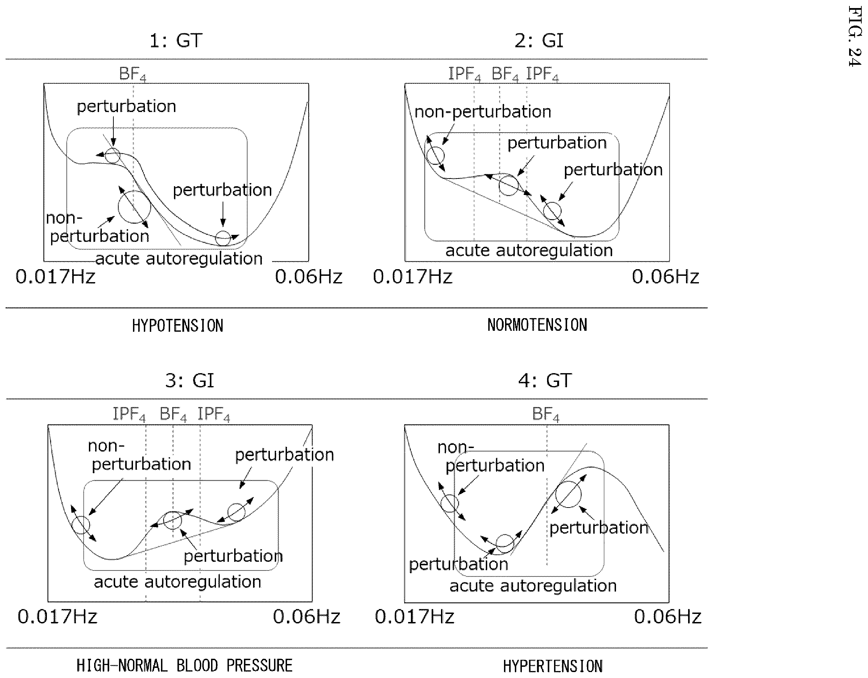

These control systems are expressed by a double-well potential system model using fitted curves represented by n-th degree polynomials, as illustrated in FIG. 23. FIG. 24 is a chart illustrating a relationship between the degree of functional enhancement in the double-well potential system model and GT (GI). In the hypotensive state represented by "1: GT" in FIG. 24, the neural control acts as an inhibitor to prevent the arterial pressure from falling any further. The fitted curve in the power spectrum plot becomes close to 1/f2 type fluctuation, and a boundary frequency (BF₄), where the main frequency components switch from periodic components to non-periodic components, or vice versa), appears near 0.02 Hz.

The normotensive state represented by "2: GI" in FIG. 24 is a bistable system model of "non-perturbation" and "perturbation" that is due to sympathetic nervous activity, resulting in 1/f type fluctuation. Regulation by the nervous system varies relatively greatly and repeatedly alternates between states of inhibition and enhancement.

The high-normal blood pressure state and the hypertensive state, which are represented by "3: GI" and "4: GT" in FIG. 24, result in a system where an upward-sloping fitted curve occurs near 0.04 Hz, suggesting the activation of "perturbation" due to neural control.

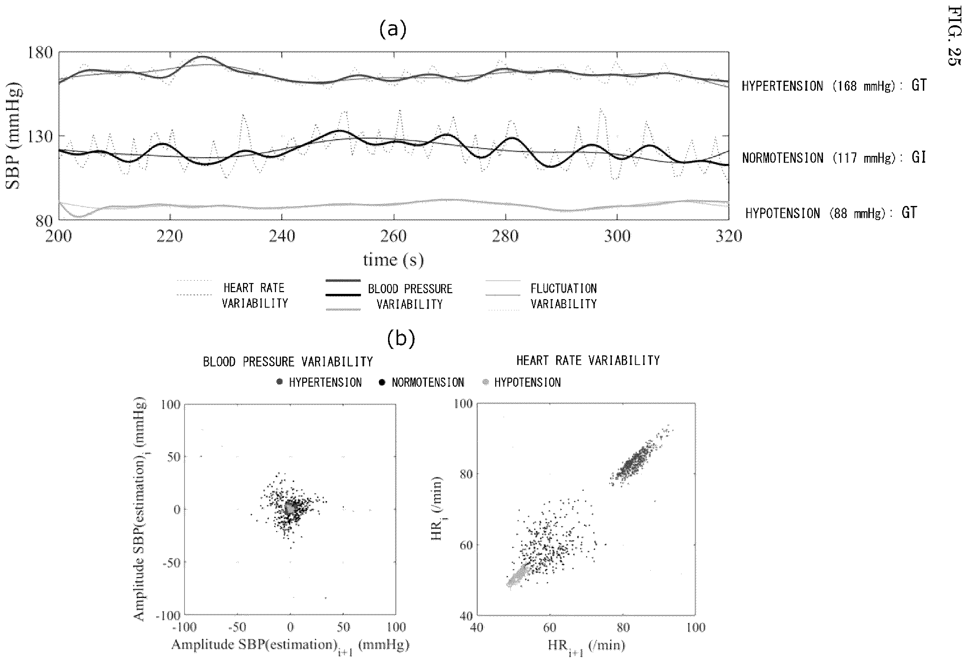

The frequency gradient (GT (GI)) according to the present invention, which reflects the control state of each system, is calculated as the frequency gradient in the range around the inflection point of the fitted curve represented by an n-th degree polynomial. This suggests the possibility that it serves as a control index of each system related to the maintenance of homeostasis of heart rate and blood pressure. Note that FIG. 25(a) is an image chart illustrating heart rate variability, blood pressure variability, and fluctuation variability in a time-series manner using analysis results and measurement data, and FIG. 25(b) is a Lorenz plot created for blood pressure variability and heart rate variability based on FIG. 25(a).

### Explanation of Reference Signs

- 1: circulatory system physiological index calculation device
- 11: biological signal receiving unit
- 12: time-series variation data calculation unit
- 13: frequency analysis unit
- 14: fitted curve computation unit
- 15: frequency gradient calculation unit
- 16: index decision unit

## Claims

1. A circulatory system physiological index calculation device that finds a circulatory system physiological index correlating with blood pressure and blood flow volume, by using at least one piece of biological signal data out of heart rate and an acoustic pulse wave that is caused by left ventricular pressure variation and obtained from a body surface, the circulatory system physiological index calculation device comprising:
a biological signal receiving unit that receives the biological signal data;
a time-series variation data calculation unit that finds time-series variation data in which information on time-series variation in the biological signal data is reflected;
a frequency analysis unit that plots and displays a power spectrum found through a frequency analysis of the time-series variation data, on a log-log graph;
a fitted curve computation unit that fits n-th degree polynomials or (n-1)-th degree polynomials to a plurality of types of analysis-target frequency bands set in a range from VLF to LF on the plot display of the power spectrum, and in the analysis-target frequency bands, finds a plurality of fitted curves represented by the n-th or (n-1)-th degree polynomials with the smallest standard deviations of residuals with respect to the plot display of the power spectrum;
a frequency gradient calculation unit that calculates a frequency gradient of a tangent at an inflection point of each of the fitted curves, and when the fitted curve has two inflection points, calculates a frequency gradient of a straight line between the two inflection points; and
an index decision unit that, regarding the tangents at the inflection points or the straight line between the inflection points, calculates standard deviations of residuals with respect to the plot display of the power spectrum, compares the standard deviations of the residuals to select the tangent or the straight line with the smallest standard deviation of the residuals, and decides the frequency gradient of the selected tangent or straight line as the circulatory system physiological index.

2. The circulatory system physiological index calculation device according to claim 1,
wherein the analysis-target frequency bands set by the fitted curve computation unit are within a range up to 0.08 Hz from a frequency of any of power spectrum calculation points that are in a 0.006 to 0.034 Hz range including 0.017 Hz, which is a fundamental frequency determined based on a basic period, the basic period being time required for acute autoregulation of blood flow to return blood flow volume to a normal level when blood pressure rises.

3. The circulatory system physiological index calculation device according to claim 1,
wherein the fitted curve computation unit determines whether a histogram of the residuals between the fitted curve drawn in each of the analysis-target frequency bands and the plot display of the power spectrum follows a normal distribution, and if the histogram does not follow the normal distribution, adjusts the analysis-target frequency band and re-finds the fitted curve.

4. The circulatory system physiological index calculation device according to claim 1, wherein the n-th degree polynomial is a quartic polynomial, and the (n-1)-th degree polynomial is a cubic polynomial.

5. The circulatory system physiological index calculation device according to claim 4,
wherein the fitted curve computation unit:
sets the analysis-target frequency bands within a range up to 0.08 Hz from a frequency of any of power spectrum calculation points that are in a range of 0.006 to 0.034 Hz, and finds a first fitted curve represented by the cubic polynomial and a second fitted curve represented by the quartic polynomial; and
finds a third fitted curve represented by the cubic polynomial in a range up to 0.08 Hz from a frequency of a lower frequency-side inflection point out of two inflection points that are found from the second fitted curve of the quartic polynomial.

6. The circulatory system physiological index calculation device according to claim 5,
wherein the index decision unit selects the straight line with the smallest standard deviation of the residuals with respect to the plot display of the power spectrum, out of a total of five straight lines consisting of:
a tangent at an inflection point of the first fitted curve;
tangents at the two inflection points of the second fitted curve;
a straight line between the two inflection points of the second fitted curve; and
a tangent at an inflection point of the third fitted curve.

7. The circulatory system physiological index calculation device according to claim 6,
wherein the index decision unit:
finds the standard deviation of the residuals regarding the tangent at the inflection points of each of the first fitted curve, the second fitted curve, and the third fitted curve in a predetermined range, of the plot display of the power spectrum, centered at the inflection point; and
regarding the second fitted curve, further finds the standard deviation of the residuals in a predetermined range, of the plot display of the power spectrum, centered at a midpoint of the straight line between the two inflection points.

8. A computer program causing a computer to function as a circulatory system physiological index calculation device that finds a circulatory system physiological index correlating with blood pressure and blood flow volume, by using at least one piece of biological signal data out of heart rate and an acoustic pulse wave that is caused by left ventricular pressure variation and obtained from a body surface, the program comprising:
a procedure for receiving the biological signal data;
a procedure for finding time-series variation data in which information on time-series variation in the biological signal data is reflected;
a procedure for plotting and displaying a power spectrum found through a frequency analysis of the time-series variation data, on a log-log graph;
a procedure for fitting n-th degree polynomials or (n-1)-th degree polynomials to a plurality of types of analysis-target frequency bands set in a range from VLF to LF on the plot display of the power spectrum, and in the analysis-target frequency bands, finding a plurality of fitted curves represented by the n-th or (n-1)-th degree polynomials with the smallest standard deviations of residuals with respect to the plot display of the power spectrum;
a procedure for calculating a frequency gradient of a tangent at an inflection point of each of the fitted curves, and when the fitted curve has two inflection points, calculating a frequency gradient of a straight line between the two inflection points; and
a procedure for, regarding the tangents at the inflection points or the straight line between the inflection points, calculating standard deviations of residuals with respect to the plot display of the power spectrum, comparing the standard deviations of the residuals to select the tangent or the straight line with the smallest standard deviation of the residuals, and deciding the frequency gradient of the selected tangent or straight line as the circulatory system physiological index.

9. The computer program according to claim 8,
wherein the analysis-target frequency bands are set within a range up to 0.08 Hz from a frequency of any of power spectrum calculation points that are in a 0.006 to 0.034 Hz range including 0.017 Hz, which is a fundamental frequency determined based on a basic period, the basic period being time required for acute autoregulation of blood flow to return blood flow volume to a normal level when blood pressure rises.

10. The computer program according to claim 9,
wherein it is determined whether a histogram of the residuals between the fitted curve drawn in each of the analysis-target frequency bands and the plot display of the power spectrum follows a normal distribution, and if the histogram does not follow the normal distribution, the analysis-target frequency band is adjusted and the fitted curve is re-found.

11. The computer program according to claim 8,
wherein the n-th degree polynomial is a quartic polynomial, and the (n-1)-th degree polynomial is a cubic polynomial.

12. The computer program according to claim 11,
wherein the analysis-target frequency bands are set within a range up to 0.08 Hz from a frequency of any of power spectrum calculation points that are in a range of 0.006 to 0.034 Hz, and a first fitted curve represented by the cubic polynomial and a second fitted curve represented by the quartic polynomial are found, and
a third fitted curve represented by the cubic polynomial is found in a range up to 0.08 Hz from a frequency of a lower frequency-side inflection point out of two inflection points that are found from the second fitted curve represented by the quartic polynomial.

13. The computer program according to claim 12,
wherein the straight line with the smallest standard deviation of the residuals with respect to the plot display of the power spectrum is selected, out of a total of five straight lines consisting of:
a tangent at an inflection point of the first fitted curve;
tangents at the two inflection points of the second fitted curve;
a straight line between the two inflection points of the second fitted curve; and
a tangent at an inflection point of the third fitted curve, and
a frequency gradient of the selected tangent or straight line is decided as the circulatory system physiological index.

14. The computer program according to claim 13,
wherein the standard deviation of the residuals regarding the tangent at the inflection point of each of the first fitted curve, the second fitted curve, and the third fitted curve is found in a predetermined range, of the plot display of the power spectrum, centered at the inflection point; and
wherein, regarding the second fitted curve, the standard deviation of the residuals is found in a predetermined range, of the plot display of the power spectrum, centered at a midpoint of the straight line between the two inflection points.

15. A computer-readable recording medium in which the computer program according to claim 8 is recorded.
